# EUROPEAN PATENT APPLICATION

(11) **EP 4 624 577 A1**
(43) Date of publication of application: **01.10.2025**
(21) Application number: 24167684.0
(22) Date of filing: 28.03.2024
(51) Int. Cl.: C12N 15/115, G01N 33/574, G01N 33/543

(54) **MULTIFUNCTIONAL SYSTEM**

(71) Applicant: Ospedale San Raffaele S.r.l., 20132 Milano (IT); Alma Mater Studiorum - Università di Bologna, 40126 Bologna (IT)
(72) Inventor: Comes Franchini, Mauro, 40126 Bologna (BO) (IT); Locatelli, Erica, 40126 Bologna (BO) (IT); Tortorella, Silvia, 40126 Bologna (BO) (IT); Alfano, Massimo, 20132 Milano (MI) (IT); Venegoni, Chiara, 20132 Milano (MI) (IT); Locatelli, Irene, 20132 Milano (MI) (IT)
(74) Representative: Pace Napoleone, Maria

(57) **Abstract**

The present invention is related to a multifunctional system bearing a ligand capable of recognizing tumor cells or tumor-associated or inflammation-associated component and linked to a metal nanoparticle. More specifically, the multifunctional system of the invention comprises a nucleotide aptamer or a variant thereof that binds to the alpha 5 subunit of integrin and a metal nanoparticle, wherein said aptamer optionally comprises at least one modification capable of enhancing nuclease resistance thus providing a molecule with high stability for example in human urine.

## Description

### FIELD OF THE INVENTION

The present invention is related to a technological platform for diagnostic, therapeutic or theragnostic applications. The invention is related to a multifunctional system bearing a ligand capable of recognizing tumor cells or tumor-associated or inflammation-associated component and linked to a metal nanoparticle. More specifically, the multifunctional system of the invention comprises a nucleotide aptamer or a variant thereof that binds to the alpha 5 subunit of integrin and a metal nanoparticle, wherein said aptamer optionally comprises at least one modification capable of enhancing nuclease resistance thus providing a molecule with high stability for example in human urine.

### BACKGROUND OF THE INVENTION

Bladder cancer (BC) is the tenth most common cancer worldwide, with more than half million new diagnoses and 200,000 death every year¹. Approximately 75% of patients who are first diagnosed with BC have tumors confined in the urothelium (in situ carcinoma-Cis, and Ta) or in the lamina propria (T1), and are classified as non-muscle invasive bladder cancer (NMIBC)². Guidelines for the clinical management of NMIBC involve endoscopic transurethral resection of bladder tumor (TURBT) followed by intravesical instillation of adjuvant therapy³. Unfortunately, BC patients with NMIBC often have very high tumor recurrence rate. Even the use of photodynamic diagnosis (performed using blue light after preoperative intravesical instillation of hexaminolaevulinic acid), followed by intravesical instillation of Mytomicin C could not reduce the frequency of NMIBC relapse, which stands at 60% 3 years post TURBT⁴. Hence, more than 200,000 BC patients each year are expected to have NMIBC relapse. The relapses often require multiple intervention sessions/cycles and follow-ups in order to eradicate the tumor. This, coupled with the high cost involved in TURBT⁵, results in BC becoming the most expensive malignancy to treat over the lifetime of the patients, with an annual expenditure of USD 10 billion^{6,7}. Consequently, reducing NMIBC relapse rate is viewed as a priority to improve the quality of life and reduce the economic burden of patients and their immediate caregivers.

To improve the success of the clinical management of NMIBC, two limitations need to be overcome. The first is technological limitation. Photodynamic diagnosis is ineffective for the detection of tumor smaller than 1 mm. This problem becomes more severe when diagnosing high-grade bladder C is due to its velvet-like appearance and with few neoplastic cells in a reddish area that is indistinguishable from inflammation. For lesions <1 mm in size, Cis cannot be diagnosed using only imaging methods, such as CT urography, IVU, US, mpMRI and the recently described high-frequency microultrasound, which although capable of discriminating between NMIBC and MIBC, can only detect tumors larger than 5 mm^{8,9}. Even cystoscopy, which includes photodynamic diagnosis, has limited diagnostic utility when it comes to Cis detection; in fact, during cystoscopy and TURBT, several biopsies from suspicious urothelium are usually performed to detect and diagnose Cis on surgical tissue specimens¹⁰. The technological limitation preventing the recognition of small lesions results in residual high-grade disease in 40% of patients after their first TURBT¹¹. The second limitation is due to the chemoresistance of residual NMIBC, with the intravesical instillation of adjuvant therapy failing in about 50% of patients³.

The present inventors have recently characterized a preclinical model of orthotopic BC whose neoplastic cells express integrin α5β1, an integrin that is also expressed in 75% of human bladder Cis¹². Using this model, they delivered a solution that allows the detection of bladder cancer lesions smaller than half millimetre¹². The solution comprised the intravesical instillation of urine-stable targeted gold nanorods (GNRs) against the integrin α5β1, and the diagnosis of BC lesions smaller than 0.5 mm expressing the integrin α5β1. This was delivered by exploiting the photoacoustic properties of GNRs using photoacoustic imaging; a diagnostic imaging modality that provides spatial resolution in the range of 50-100 microns¹³. This solution comprised the use of GNRs coated with thiolated chitosan (GNRs@Chit) and functionalized with the peptide *Iso4* (head-to-tail cyclized c(CphgisoDGRG), selective for the integrin α5β1 ¹². This approach demonstrated the feasibility of using GNRs@Chit-Iso4 as a urine-stable nanosystem able to maintain its chemical-physical properties and binding activity for bladder tumor cells α5β1 integrin-positive. The GNRs@Chit-Iso4 is instilled in the bladder under ultrasound-mediated shaking to avoid sedimentation in the lower part of the organ¹².

### SUMMARY OF THE INVENTION

The present invention provides a novel multifunctional system that maintains chemical-physical properties in the presence of urine and binding capacity to bladder tumor cells positive for the integrin α5β1. In the multifunctional system of the invention GNRs coated with thiolated chitosan (GNRs@Chit) have been engineered with a novel stable aptamer resistant to nucleases, highly present in the urine¹⁴. The aptamer has been obtained through optimization of a previously generated unmodified RNA aptamer targeting integrin α5β1¹⁵ by eliminating dispensable nucleotides of its sequence and replacing all the pyrimidines with 2'Fluoropyrimidines (2'F-Py) to improve its stability. The shorter aptamer, named Apt-Itg, maintains the targeting efficacy of the parental aptamer.

The novel nanosystem GNRs@Chit-Apt-Itg is stable in the urinary environment, does not require ultrasound-assisted shaking because has high polydispersity index and uniform particle size distribution. In addition, the synthesis of the Apt-Itg is highly reproducible with no racemization issues, it is cheaper than Iso4 or other form of ligands as antibodies, because the quality control of the production requires a limited number of tests (i.e. polymerase chain reaction) and does not involve the use of animals that makes the production expensive and time consuming¹⁶. The chemical synthesis of the aptamer is more cost-effective than cyclic peptides or antibodies (low cost/high yield and small batch-to-batch variation) for industrial scale up, and allows easier conjugation to nanovectors¹⁷, including GNRs@Chit.

It is an object of the invention a multifunctional system comprising a nucleotide aptamer or a variant thereof that binds to the alpha 5 subunit of integrin and a metal nanoparticle, wherein said aptamer comprises or consists of a nucleotide sequence that is at least 70% identical of 5'-GCGGACGGACAGAGAGUGCAACCUGCCGUGCCGC-3' (SEQ ID No: 1) or functional fragment thereof and optionally comprises at least one modification capable of enhancing nuclease resistance.

Preferably, the at least one modification capable of enhancing nuclease resistance is fluorination in the 2'-position of a pyrimidine nucleotide.

More preferably the aptamer or variant thereof or functional fragments thereof comprises or consists of a nucleotide sequence that is at least 70% identical of: 5'-G(2'F-C)GGA(2'F-C)GGA(2'F-C)AGAGAG(2'F-U)G(2'F-C)AA(2'F-C)(2'F-C)(2'F-U)G(2'F-C)(2'F-C)G(2'F-U)G(2'F-C)(2'F-C)G(2'F-C)-3' (SEQ ID No:2).

Even more preferably, the aptamer or variant thereof or functional fragments thereof is functionalized at its 5' end with a thiol group, preferably with a C6-C12 aliphatic-thio molecule, preferably wherein the aptamer comprises or consists of a nucleotide sequence that is at least 70% identical of SEQ ID No: 2 or variant thereof or functional fragments thereof and is functionalized with a C6-thiol group at its 5'end.

In a preferred embodiment of the invention the metal nanoparticle is made of gold, silver, or hybrid gold/silver, preferably gold and the metal nanoparticle has photoacoustic properties, preferably wherein the metal nanoparticle is chosen among nanosphere, nanorod, nanostar, nanocage, nanoprism or nanoshell or nanowire, nanoplate, hallow shell, preferably the metal nanoparticle is gold nanorod with a size ranging from 10 to 200 nm in length, and 2 to 50 nm in width, more preferably ranging from 20 to 100 nm in length and from 10 to 25 nm in width and the preferred aspect ratio ranges between 1.2 and 15, more preferably between from 3 and 7.

In a further preferred embodiment the metal nanoparticle is coated with a polymer functionalized with thiol groups and -NH groups, said polymer preferably being selected among thiolated chitosan, thiolated and aminated alginic acid, thiolated and aminated hyaluronic acid, or a protein preferably selected from the group consisting of albumin and gelatin, or a synthetic thiolated and aminated polymer, preferably α-thio-ω-amino polyethylene glycols.

Preferably the polymer functionalized with thiol groups and -NH groups is thiolated chitosan preferably having an average molecular weight ranging from 0.5 kDa to 1000 kDa, more preferably ranging from 50 kDa to 200 kDa, and an average deacetylation degree ranging from 75% to 100%, more preferably ranging from 85% to 95%.

Preferably, in the multifunctional system said thiol groups and -NH groups of the polymer are linked via a crosslinker to the aptamer that binds to the alpha 5 subunit of integrin and wherein:
- the crosslinker bears at least one functional group able to bind to an amino group, said functional group being preferably selected from the group consisting of N-hydroxysuccinimidyl ester group (NHS ester), an isocyanate group (-NCO), an isothiocyanate group (-NCS), a sulfo-N-hydroxysuccinimidyl ester group (sulfo-NHS ester), a maleimide group or a carboxylic acid; and/or
- the cross linker bears at least one functional group able to bind a thiol group, said functional group being preferably selected from the group consisting of: a maleimide group, a terminal vinyl group or a terminal alkyne group; and/or
- the cross linker bears at least one functional group able to bind an alkyne such as an azide; and/or
- the cross linker bear at least one functional group able to bind an azide, such as an alkyne.

Preferably the crosslinker is maleimido-PEG₁₂-NHS ester.

Preferably in the multifunctional system of the invention:
- the aptamer has SEQ ID No:2 and is preferably functionalized at its 5'end with a C6-thiol group;
- the metal nanoparticle is gold nanorod;
- the functionalized polymer is thiolated chitosan;
- the crosslinker is maleimido-PEG₁₂-NHS ester.

It is a further object of the invention a composition comprising the multifunctional system as defined above or a kit comprising single use vials containing the multifunctional system as defined above, said composition and kit further comprising at least one of the following solvents: water; physiologic solution; Dulbecco's Modified Eagle Medium (DMEM); Dulbecco's phosphate-buffered saline (DPBS), HEPES buffer, TRIS buffer, PIPES buffer each containing metal divalent ions such as Ca²⁺, Mg²⁺.

Preferably said composition or said kit further comprises at least one or more antitumoral agents preferably chosen among a chemotherapeutic agent, an immunomodulator, an immune cell.

It is a further object of the invention the multifunctional system or the composition or the kit as defined above for medical use, preferably for use in a method of diagnosis and/or treatment in vivo, more preferably for use in the diagnosis and/or treatment of solid tumors.

It is a further object of the invention the multifunctional system or the composition or the kit as defined above for use in the treatment of a tumor selected among urothelial, bladder, gastroesophageal, colorectal, pancreatic, ovarian, lung, cervix, breast and renal cancer, brain tumors, hollow organs cancer and hepatocellular carcinoma.

It is a further object of the invention the multifunctional system or the composition or the kit as defined above for use in the photothermal therapy of solid tumors, preferentially bladder cancer.

It is a further object of the invention an in-vitro diagnostic method for detecting cancer from a biological sample of a test subject, said method comprising the following steps:
a) contacting said biological sample with at least one multifunctional system of the invention or with the composition or kit as defined above, and
b) photoacoustic or ultrasound visualization of the test sample, and
c) detection of the presence of alpha 5 beta 1 integrin, and
d) evaluation by comparison with a reference sample.

It is a further object of the invention an aptamer that binds to the alpha 5 subunit of integrin comprising or consisting of a nucleotide sequence that is at least 70% identical of 5'-GCGGACGGACAGAGAGUGCAACCUGCCGUGCCGC-3' (SEQ ID No: 1) or a variant thereof or a functional fragment thereof and having at least one modification capable of enhancing nuclease resistance, preferably wherein the at least one modification capable of enhancing nuclease resistance is fluorination in the 2'-position of a pyrimidine nucleotide, preferably wherein the aptamer comprises or consists of a nucleotide sequence that is at least 70% identical of: 5'-G(2'F-C)GGA(2'F-C)GGA(2'F-C)AGAGAG(2'F-U)G(2'F-C)AA(2'F-C)(2'F-C)(2'F-U)G(2'F-C)(2'F-C)G(2'F-U)G(2'F-C)(2'F-C)G(2'F-C)-3' (SEQ ID No:2) or a variant thereof or a functional fragment thereof.

### BRIEF DESCRIPTION OF THE DRAWINGS

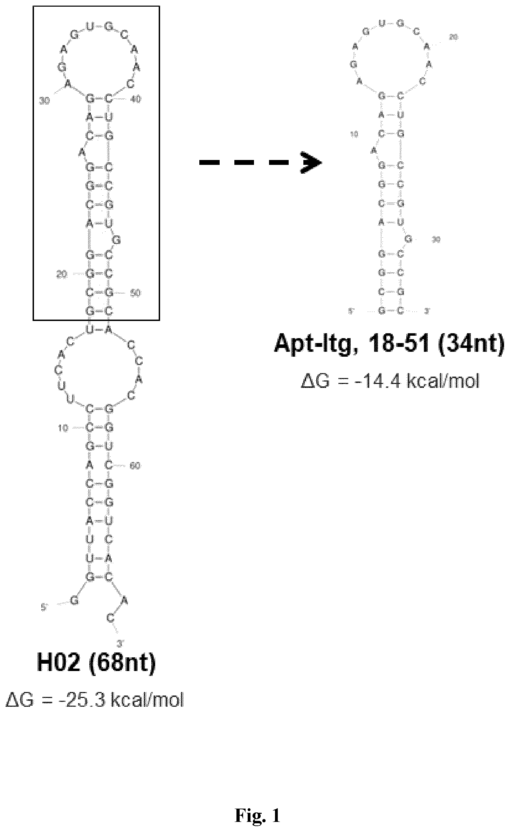

**Figure 1****. The secondary structure of H02 full-length and Apt-Itg (nucleotides from 18 to 51, boxed).** Sequences of the H02 full-length and Apt-Itg were predicted by using Mfold web server. The calculated change in Gibbs free energy (ΔG) values are reported.

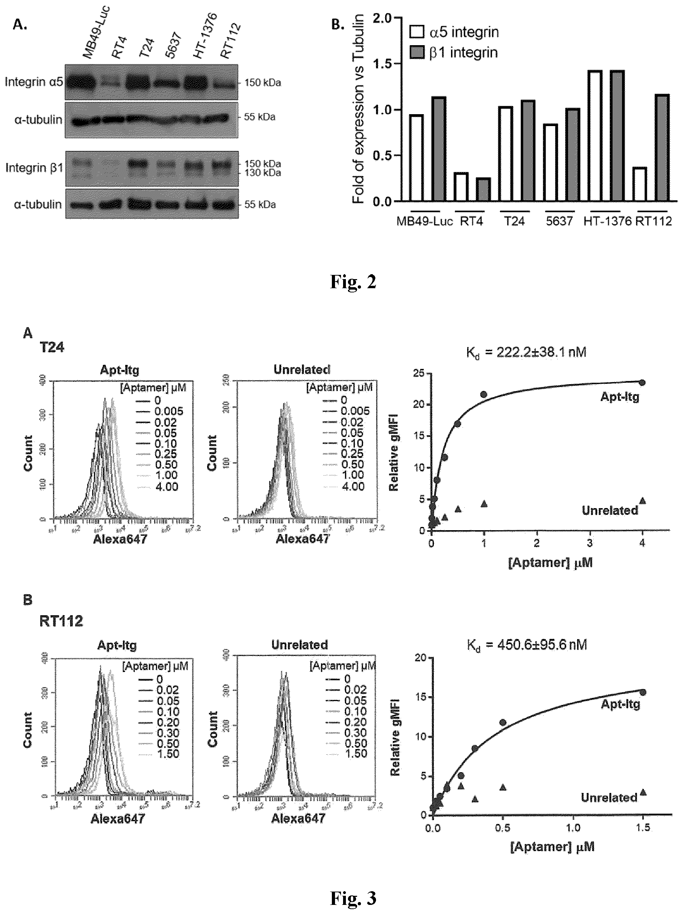

**Figure 2****. The expression of integrin a5b1 by human and murine bladder cancer cell lines.** A) Western blot analysis of the α5 and β1 chain of the integrin, and of the housekeeping protein α-tubulin. B) Relative fold of expression of integrin chains against the housekeeping protein α-tubulin.
**Figure 3****. Binding assay of Alexa 647-Apt-Itg to T24 and RT112 cells using flow cytometry.** Representative binding profiles of increasing concentrations of Alexa 647-Apt-Itg and Unrelated aptamer incubated with T24 (A) and RT112 (B) cells. Apparent dissociation curves of aptamer-cell interaction and Kd value of Apt-Itg is shown.

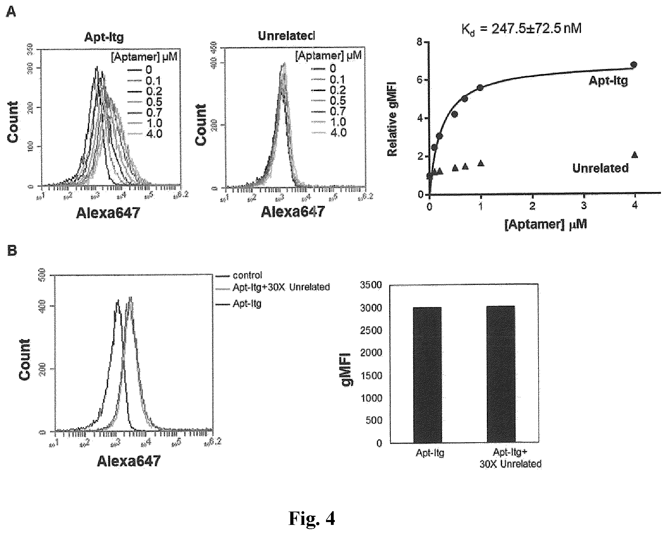

**Figure 4****. Binding assay of Alexa 647-Apt-Itg to MB49-Luc cells using flow cytometry.** (A) Representative binding profiles of increasing concentrations of Alexa 647-Apt-Itg and unrelated aptamer incubated with MB49-Luc cells (left). Apparent dissociation curve of Apt-Itg-cell interaction and Kd value are shown (right). (B) Competition binding assay by flow cytometry (left) and quantification of the gMFI (right) using 1 µM Alexa 647-Apt-Itg in the absence (blue) or in the presence (red) of 30-fold excess of unlabelled unrelated aptamer.

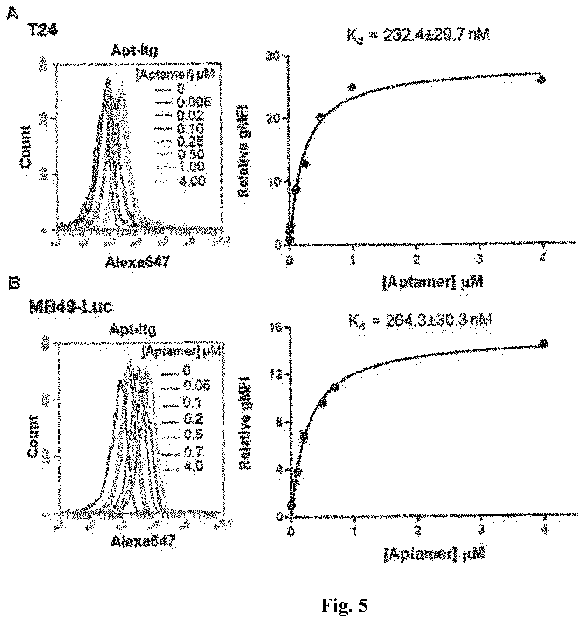

**Figure 5****. Binding assay of Alexa 647-Apt-Itg to human and murine cancer cells in the presence of divalent cations.** Representative binding profiles of increasing concentrations of Alexa 647-Apt-Itg incubated with human bladder cancer cell line T24 (A) and murine bladder cancer cell line MB49-Luc (B) cells in presence of 1 mM MgCl₂ and 0.5 mM CaCl₂. Apparent dissociation curves of aptamer-cell interaction and Kd value of Apt-Itg is shown. Data are presented as the mean ± SD.

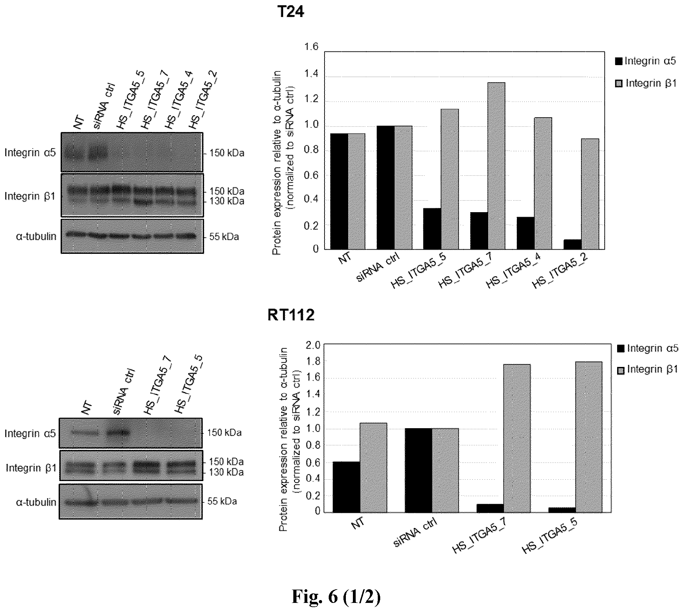

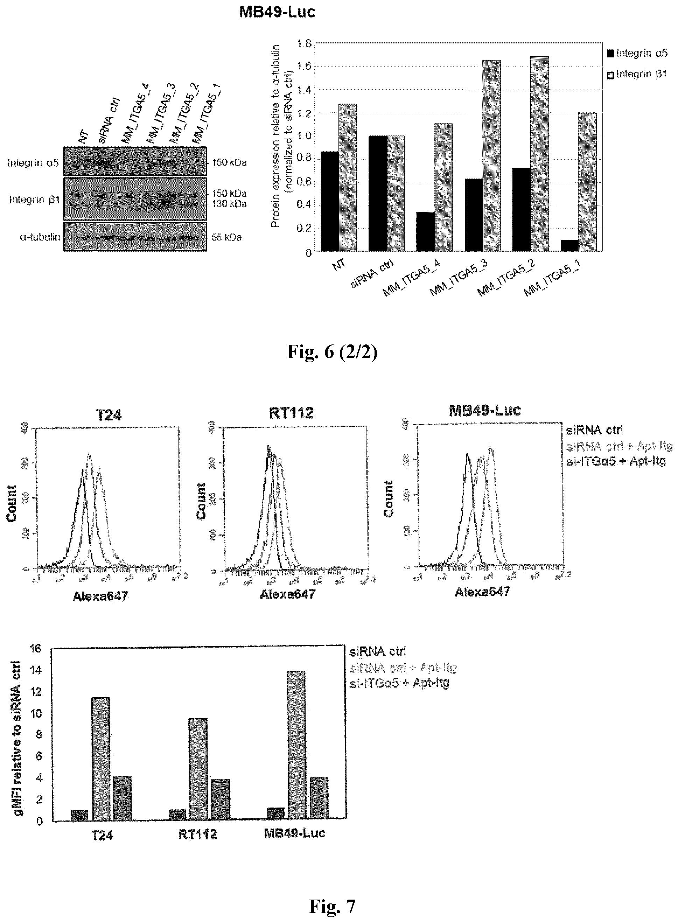

**Figure 6****. Integrin α5 silencing using siRNAs.** T24, RT112 and MB49-Luc cells were left untreated (NT) or transfected with the indicated Integrin α5 siRNAs or siRNA ctrl. At 24 h post-transfection, cells were harvested, and cell lysates prepared and immunoblotted with anti-integrin α5 or anti-integrin β1 antibody. Equal loading was confirmed by immunoblot with anti-α-tubulin antibody. The histograms indicate the integrin chains/α-tubulin ratio of the densitometric signals. Values are shown relative to siRNA ctrl, arbitrarily set to 1.
**Figure 7****. Integrin α5 silencing results in reduced Apt-Itg binding.** Binding of 1 µM Alexa647-Apt-Itg on human and murine bladder cancer cells following 24 hours-transfection with si-ITGα5 and siRNA ctrl. The histogram indicates gMFI of aptamer-treated cells normalized to the gMFI of siRNA ctrl untreated cells (black), arbitrarily set to 1.

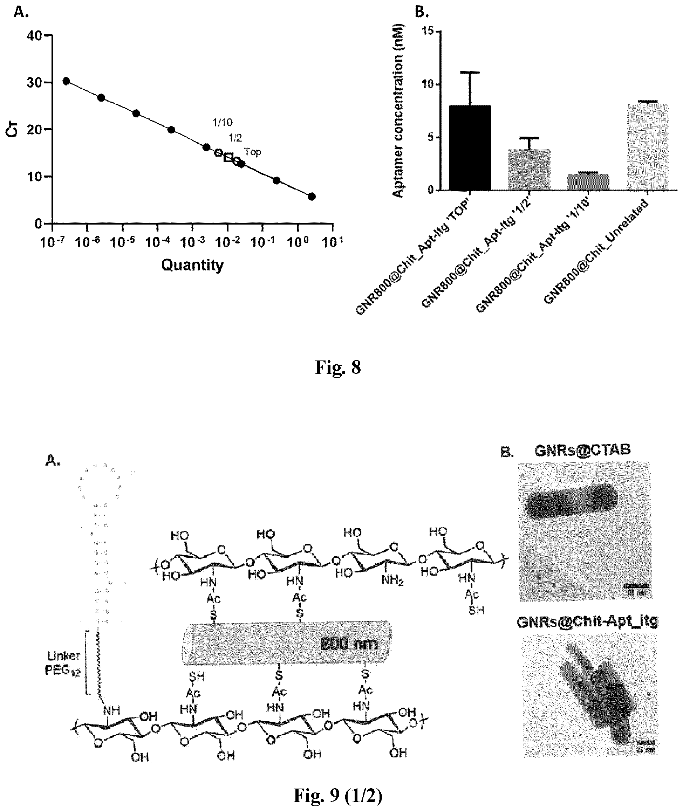

**Figure 8****. Quantification of the amount of Apt-Itg and unrelated aptamer conjugated to GNRs@Chit.** PCR calibration curve with unconjugated Apt-Itg (red); sample, green (A), and quantification of the Apt-Itg and unrelated aptamer loaded onto GNRs@Chitosan (B). Data shown are mean ± SD of three independent experiments.

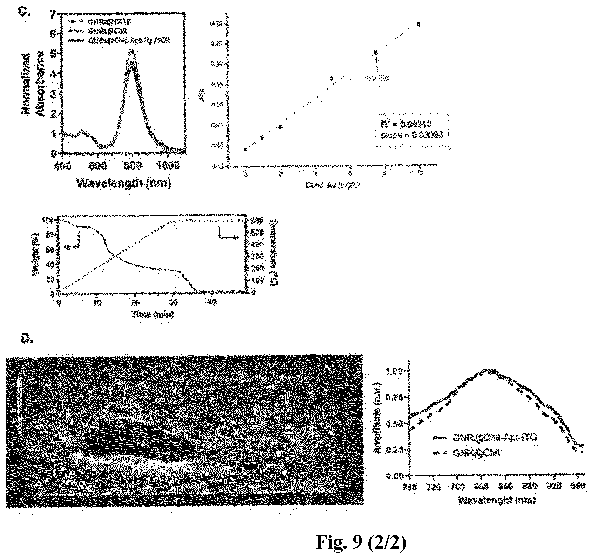

**Figure 9****. Characterization of the final nanosystem, GNRs@Chit-Apt_Itg.** A) Scheme representative of the aptamer Apt-Itg conjugated to the GNRs@Chit via PEG₁₂ linker. B) TEM images of nanoparticles. C) UV-Vis spectra of GNRs@CTAB, GNRs@Chit and GNRs@Chit-Apt_Itg/SCR showing no significant differences in the absorption spectrum of GNRs after surface modification (SCR indicates a scrambled sequence used as control); FAAS analysis of GNRs@Chit-Apt_Itg with calibration line; thermogravimetric analysis of GNRs@Chit in N2 and air atmosphere (above 600 °C). D) Photoacoustic imaging of agar drop containing GNR@Chit-Apt-ITG (6 µg Au; 30 nmol Au, *red*), with the echogenic signal (*gray*) generated by the slime in which the agar drop is embedded. The light blue region of interest in panel A delineates the PA signal of GNRs from which the photoacoustic spectra was derived (B; normalized PA spectra of GNRs@Chit-Apt-Itg and unconjugated GNRs@Chit-*Iso4*, embedded in agar drops). The PA images were acquired and spectrally unmixed with VevoLab software to separate the contribution of the slime and GNRs (the *red signal* corresponds to the PA signal of GNRs).

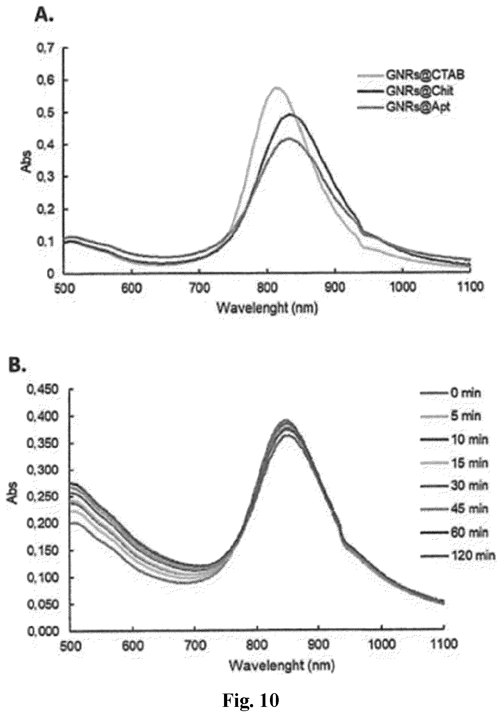

**Figure 10****. GNRs@Chit-Apt-Itg stability in human urine.** A) VIS-NIR spectra of GNRs@Chit-Apt compared to the VIS-NIR spectra of GNRs@CTAB and GNRs@Chit. B) VIS-NIR spectra and absorption intensity of GNRs@Chit-Apt in the presence of human urine over time.

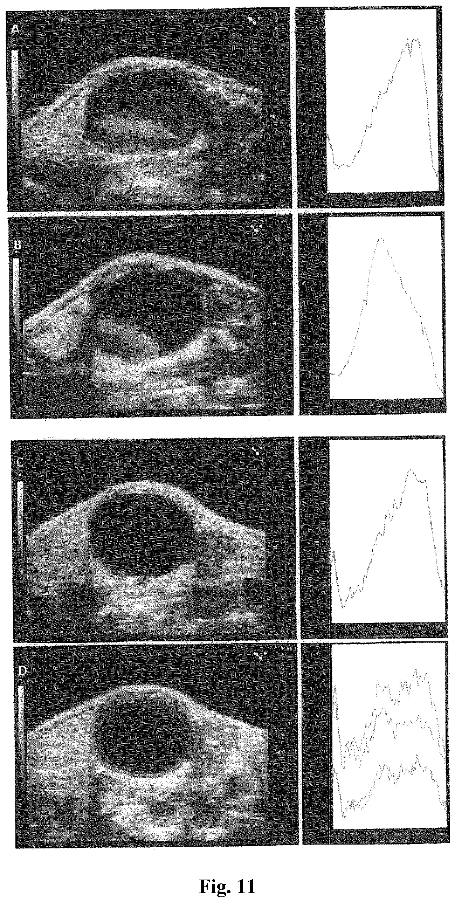

**Figure 11****. The photoacoustic imaging of GNRs@chit-Apt-Itg is specific for the tumor mass.** Axial frame of a murine bladder with an US recognizable tumor nine days after intravesical instillation of MB49-Luc cells, with a region of interest in which a photoacoustic spectrum has been analysed, before (A) and 15 minutes after (B) the intravesical instillation of GNRs@Chit-Apt-Itg. Axial frame of a murine bladder with a region of interest in which a photoacoustic spectrum has been analysed, before (C) and 15 minutes after (D) the intravesical instillation of GNRs@Chit-Apt-Itg. GNRs@Chit-Apt-Itg; instilled 100 ul (100 uM Au) with the "TOP" amount of aptamer.

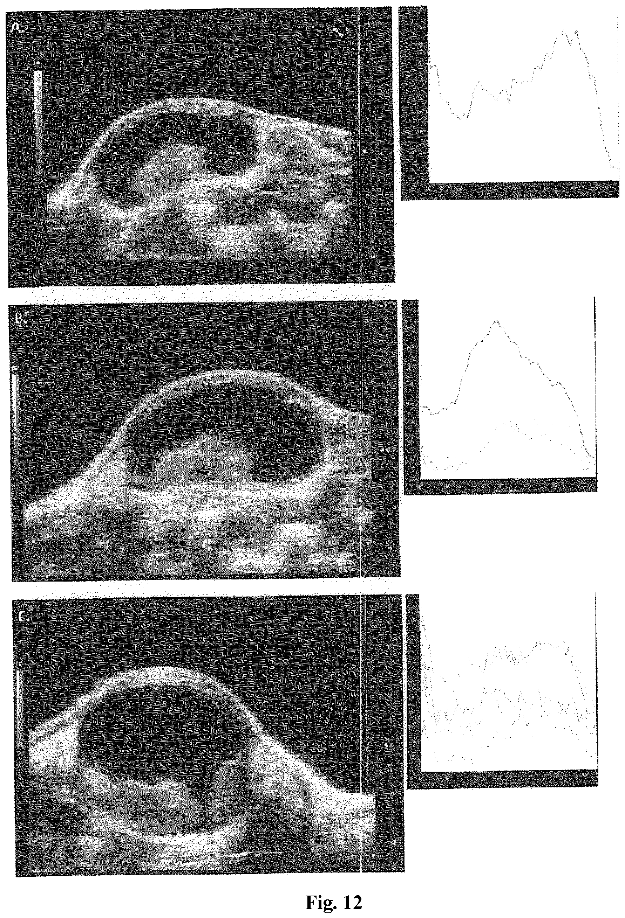

**Figure 12****. The photoacoustic signalling of GNRs is lost 18 hours after intravesical instillation of GNRs@chit-Apt-Itg.** Axial frame of a murine bladder with an US recognizable tumor nine days after intravesical instillation of MB49-Luc cells, with a region of interest in which a photoacoustic spectrum has been analysed, showing a PA spectra ascribed to oxygenated blood. Fifteen minutes after the intravesical instillation of GNR@Chit-Apt-Itg the correct PA spectra of the GNRs was identified on top and lateral sides of the tumor mass, but not in the surrounding non-neoplastic utrothelium (B). Eighteen hours later the PA signal of the GNRs was not detectable, and tumor was growth on the lateral sides of the mass identified the day before. The amount of intravesically instilled of GNR@Chit-Apt-Itg was 100 ul at concentration of 100 uM Au, loaded with the TOP amount of aptamer.

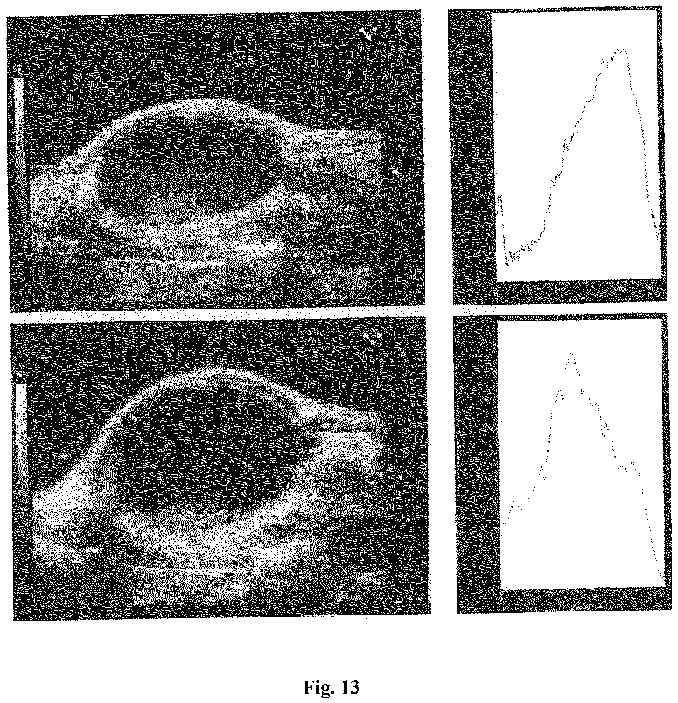

**Figure 13****. Tumor mass is recognized by GNRs@chit-Apt-Itg loaded with lower amount of Apt-Itg.** Axial frame of a murine bladder with an US recognizable tumor nine days after intravesical instillation of MB49-Luc cells, with a region of interest in which a photoacoustic spectrum has been analysed, before (A) and 15 minutes after (B) the intravesical instillation of GNRs@Chit-Apt-Itg. GNRs@Chit-Apt-Itg. GNRs@Chit-Apt-Itg; instilled 100 ul (100 uM Au) with the "1/10" amount of aptamer.

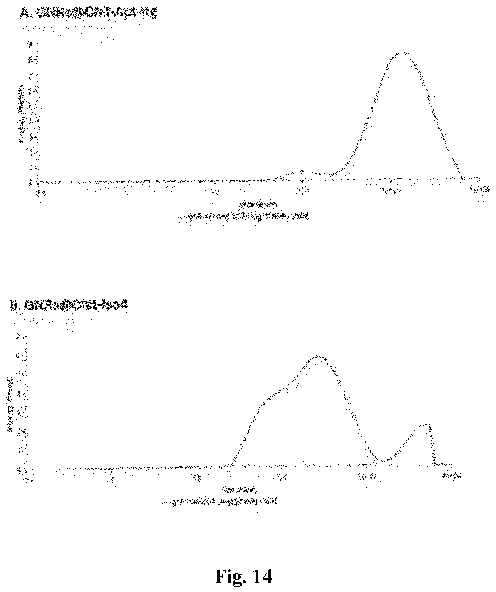

**Figure 14****. The hydrodynamic range of the targeted GNRs.** Histograms showing the hydrodynamic range (Z-average, expressed in nm) of A) GNRs@Chit-Apt-Itg (loaded with TOP amount of Apt-Itg) and B) GNRs@Chit-Iso4. Histograms shows the mean of measurements performed in triplicate.

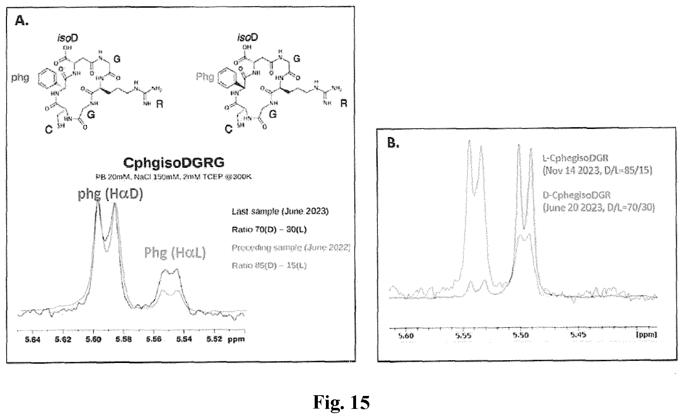

**Figure 15****. Characterization of peptide Iso4 by NMR.** A) NMR spectrum of Iso4 peptide synthetized as D-Iso4, giving rise to a mixture of D and L isoforms, with a relative amount that is variable between preparations (two different synthesis are shown). B) The D and L isoforms are present also when the peptide was synthesized as L-Iso4.

### DETAILED DESCRIPTION OF THE INVENTION

The aim of the invention is to provide a technological platform for diagnostic, therapeutic or theragnostic applications. While the invention is susceptible to various alternative modifications, some preferred embodiments are described below in detail; said embodiments are example and shouldn't be intended as limiting of the scope of the invention to the specific alternative.

It is therefore object of the present invention the development of a multifunctional system, preferably metal nanostructures or nanoparticles bearing a ligand capable of recognizing a tumor-associated or inflammation-associated component.

It is an object of the invention a multifunctional system comprising a nucleotide aptamer or a variant thereof that binds to the alpha 5 subunit of integrin and a metal nanoparticle, wherein said aptamer comprises or consists of a nucleotide sequence that is at least 70% identical of 5'-GCGGACGGACAGAGAGUGCAACCUGCCGUGCCGC-3' (SEQ ID No: 1) or functional fragment thereof and optionally comprises at least one modification capable of enhancing nuclease resistance.

Preferably, the at least one modification capable of enhancing nuclease resistance is fluorination in the 2'-position of a pyrimidine nucleotide.

More preferably the aptamer or variant thereof or functional fragments thereof comprises or consists of a nucleotide sequence that is at least 70% identical of: 5'-G(2'F-C)GGA(2'F-C)GGA(2'F-C)AGAGAG(2'F-U)G(2'F-C)AA(2'F-C)(2'F-C)(2'F-U)G(2'F-C)(2'F-C)G(2'F-U)G(2'F-C)(2'F-C)G(2'F-C)-3' (SEQ ID No:2).

Even more preferably, the aptamer or variant thereof or functional fragments thereof is functionalized at its 5' end with a thiol group, preferably with a C6-C12 aliphatic-thio molecule, preferably wherein the aptamer comprises or consists of a nucleotide sequence that is at least 70% identical of SEQ ID No: 2 or variant thereof or functional fragments thereof and is functionalized with a C6-thiol group at its 5'end.

In a preferred embodiment of the invention the metal nanoparticle is made of gold, silver, or hybrid gold/silver, preferably gold and the metal nanoparticle has photoacoustic properties, preferably wherein the metal nanoparticle is chosen among nanosphere, nanorod, nanostar, nanocage, nanoprism or nanoshell or nanowire, nanoplate, hallow shell, preferably the metal nanoparticle is gold nanorod with a size ranging from 10 to 200 nm in length, and 2 to 50 nm in width, more preferably ranging from 20 to 100 nm in length and from 10 to 25 nm in width and the preferred aspect ratio ranges between 1.2 and 15, more preferably between from 3 and 7.

In a further preferred embodiment the metal nanoparticle is coated with a polymer functionalized with thiol groups and -NH groups, said polymer preferably being selected among thiolated chitosan, thiolated and aminated alginic acid, thiolated and aminated hyaluronic acid, or a protein preferably selected from the group consisting of albumin and gelatin, or a synthetic thiolated and aminated polymer, preferably α-thio-ω-amino polyethylene glycols.

Preferably the polymer functionalized with thiol groups and -NH groups is thiolated chitosan preferably having an average molecular weight ranging from 0.5 kDa to 1000 kDa, more preferably ranging from 50 kDa to 200 kDa, and an average deacetylation degree ranging from 75% to 100%, more preferably ranging from 85% to 95%.

Preferably, in the multifunctional system said thiol groups and -NH groups of the polymer are linked via a crosslinker to the aptamer that binds to the alpha 5 subunit of integrin and wherein:
- the crosslinker bears at least one functional group able to bind to an amino group, said functional group being preferably selected from the group consisting of N-hydroxysuccinimidyl ester group (NHS ester), an isocyanate group (-NCO), an isothiocyanate group (-NCS), a sulfo-N-hydroxysuccinimidyl ester group (sulfo-NHS ester), a maleimide group or a carboxylic acid; and/or
- the cross linker bears at least one functional group able to bind a thiol group, said functional group being preferably selected from the group consisting of: a maleimide group, a terminal vinyl group or a terminal alkyne group; and/or
- the cross linker bears at least one functional group able to bind an alkyne such as an azide; and/or
- the cross linker bear at least one functional group able to bind an azide, such as an alkyne.

Preferably the crosslinker is maleimido-PEG₁₂-NHS ester.

Preferably in the multifunctional system of the invention:
- the aptamer has SEQ ID No:2 and is preferably functionalized at its 5'end with a C6-thiol group;
- the metal nanoparticle is gold nanorod;
- the functionalized polymer is thiolated chitosan;
- the crosslinker is maleimido-PEG₁₂-NHS ester.

It is a further object of the invention a composition comprising the multifunctional system as defined above or a kit comprising single use vials containing the multifunctional system as defined above, said composition and kit further comprising at least one of the following solvents: water; physiologic solution; Dulbecco's Modified Eagle Medium (DMEM); Dulbecco's phosphate-buffered saline (DPBS), HEPES buffer, TRIS buffer, PIPES buffer each containing metal divalent ions such as Ca²⁺, Mg²⁺.

Preferably said composition or said kit further comprises at least one or more antitumoral agents preferably chosen among a chemotherapeutic agent, an immunomodulator, an immune cell.

It is a further object of the invention the multifunctional system or the composition or the kit as defined above for medical use, preferably for use in a method of diagnosis and/or treatment in vivo, more preferably for use in the diagnosis and/or treatment of solid tumors.

It is a further object of the invention the multifunctional system or the composition or the kit as defined above for use in the treatment of a tumor selected among urothelial, bladder, gastroesophageal, colorectal, pancreatic, ovarian, lung, cervix, breast and renal cancer, brain tumors, hollow organs cancer and hepatocellular carcinoma.

It is a further object of the invention the multifunctional system or the composition or the kit as defined above for use in the photothermal therapy of solid tumors, preferentially bladder cancer.

It is a further object of the invention an in-vitro diagnostic method for detecting cancer from a biological sample of a test subject, said method comprising the following steps:
e) contacting said biological sample with at least one multifunctional system of the invention or with the composition or kit as defined above, and
f) photoacoustic or ultrasound visualization of the test sample, and
g) detection of the presence of alpha 5 beta 1 integrin, and
h) evaluation by comparison with a reference sample.

It is a further object of the invention an aptamer that binds to the alpha 5 subunit of integrin comprising or consisting of a nucleotide sequence that is at least 70% identical of 5'-GCGGACGGACAGAGAGUGCAACCUGCCGUGCCGC-3' (SEQ ID No: 1) or a variant thereof or a functional fragment thereof and having at least one modification capable of enhancing nuclease resistance, preferably wherein the at least one modification capable of enhancing nuclease resistance is fluorination in the 2'-position of a pyrimidine nucleotide, preferably wherein the aptamer comprises or consists of a nucleotide sequence that is at least 70% identical of: 5'-G(2'F-C)GGA(2'F-C)GGA(2'F-C)AGAGAG(2'F-U)G(2'F-C)AA(2'F-C)(2'F-C)(2'F-U)G(2'F-C)(2'F-C)G(2'F-U)G(2'F-C)(2'F-C)G(2'F-C)-3' (SEQ ID No:2) or a variant thereof or a functional fragment thereof. Preferably said aptamer is for medical use, even more preferably is for use for use in a method of diagnosis and/or treatment in vivo, more preferably for use in the diagnosis and/or treatment of solid tumors, even more preferably for use in the diagnosis and/or treatment of a tumor selected among urothelial, bladder, gastroesophageal, colorectal, pancreatic, ovarian, lung, cervix, breast and renal cancer, brain tumors, hollow organs cancer and hepatocellular carcinoma.

As used herein, the term "multifunctional system" indicates the complex, functionalized system of the invention, which can be also referred as "targeted nanoparticle" or "nanovector", and includes at least the aptamer of the invention and the metal nanoparticle, preferably the aptamer, the coated metal nanoparticle and the heterobifunctional crosslinker.

In the context of the present invention, the term "variant" refers to longer or shorter polynucleotide sequences and/or polynucleotides having, for example, an identity percentage of at least 41%, 50%, 60%, 65%, 70% or 75%, more preferably of at least 85%, for example of at least 90%, and more preferably of at least 95% or 100% with the sequences described herein or with the complementary sequence thereof or with the corresponding DNA or RNA sequence thereof.

The term "variant" and the term "polynucleotide" also comprise modified synthetic oligonucleotides. These modified synthetic oligonucleotides are preferably locked nucleic acids (LNA), phosphoro-thiolated oligo, or methylated oligo, morpholines, 2'-O-methyl or 2'-O-methoxyethyl oligonucleotides and cholesterol-conjugated 2'-O-methyl modified oligonucleotides (antagomirs). The term "variant" can also include nucleotide analogs, i.e., a ribonucleotide or a natural deoxyribonucleotide substituted by a non-natural nucleotide, and nucleic acids which can be generated by mutating one or more nucleotides or amino acids in the sequences thereof, or in the precursor sequences or equivalent. The term "variant" also comprises at least one functional fragment of the polynucleotide.

In the context of the present invention, "functional" means that it maintains the functional features of the aptamers from which they derive, e.g., the ability to bind to the alpha 5 subunit of integrin.

The term "variant" as used herein refers to compounds which have a similar but not identical chemical formula and share the same or substantially similar function as the compound with the similar chemical formula. In some embodiments, the analog is a mutant, variant, or modified sequence with respect to the unmodified or wild-type sequence on which it relies. In some embodiments, the analog is a functional fragment of any one of the nucleic acid sequences described herein. In some embodiments, the analog is a salt of any one of the nucleic acid sequences described herein. In such embodiments, the analog can retain about 99%, 98%, 97%, 96%, 95%, 94%, 93%, 92%, 91%, 90%, 85%, 80%, 75%, 70% or less biological activity with respect to the wild or natural-type sequences on which it is based.

Preferably, the aptamer or the variant thereof according to the invention selectively binds to the alpha 5 subunit of integrin.

The high flexibility of the molecule also makes it suitable for further chemical modifications aimed at improving the pharmacodynamic and pharmacokinetic properties thereof, as well as for the use thereof in molecular imaging techniques. Possible chemical modifications include the functionalization at its 5'end with an aliphatic chain, preferably of 6 to 12 methylene unit, comprising a thiol group. According to the invention an aptamer thereof functionalized at its 5' end with a thiol group, preferably with a C6-C12 aliphatic-thio molecule, is an aptamer linked at its 5'end to an aliphatic chain of the indicated length also bearing a thiol group.

According to the invention the aptamer may comprise at least one modified pyrimidine nucleotide. According to the invention the modified pyrimidine may be any modified pyrimidine known to one skilled in the art. It may be for example a chemically modified pyrimidine and/or a substituted pyrimidine, for example at the position 2'. Preferably the pyrimidine nucleotide may be modified to be a 2'-fluoro pyrimidine. Said modifications are known as enhancing nuclease resistance of the aptamer, thus increasing its stability in the in vivo setting, in particular in urine. The sequences of nucleic acids, protein or other agents of the present description can be administered, inter alia, as pharmaceutically acceptable salts, esters or amides. The term "salts" refers to inorganic and organic salts of the compounds of the present description. The salts can be prepared in situ during the final isolation and purification of a compound, or by separately reacting a purified compound in the free base or acid form thereof with a suitable organic or inorganic base or acid and isolating the salt thus formed. The representative salts include hydrobromide, hydrochloride, sulfate, bisulfate, nitrate, acetate, oxalate, palmitate, stearate, laurate, borate, benzoate, lactate, phosphate, tosylate, citrate, maleate, fumarate, succinate, tartrate, naphthylate, mesuco, gluco, lactobionate and lauryl sulfonate salts, and the like. The salts can include cations based on alkali and alkali earth metals, such as sodium, lithium, potassium, calcium, magnesium, and the like, as well as non-toxic ammonium, quaternary ammonium, and amine cations including, but not limited to, ammonium, tetramethylammonium, tetraethylammonium, methylamine, dimethylamine, trimethylamine, triethylamine, ethylamine, and the like; see S. M. Berge, et al., J Pharm Sci, 66: 1-19 (1977), describing the saline forms of nucleic acids and incorporated by reference in the entirety thereof. Non limitative examples of the cross-linker (also referred as crosslinker, cross linker or linker) according to the invention are reported below.

Succinimidyl-4-(N-maleimidomethyl)cyclohexane-1-carboxylate (SMCC) is a non-cleavable and membrane permeable crosslinker, with the following structure:

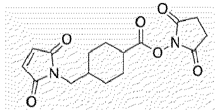

SMCC contains an amine-reactive N -hydroxysuccinimide (NHS ester) and a sulfhydryl-reactive maleimide group. NHS esters react with primary amines at pH 7-9 to form stable amide bonds. Maleimides react with sulfhydryl groups at pH 6.5-7.5 to form stable thioether bonds.

Sulfo-SMCC (sulfosuccinimidyl 4-(N-maleimidomethyl)cyclohexane-1-carboxylate) corresponds to the compound having the chemical structure indicated below:

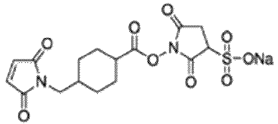

The maleimide groups of SMCC and Sulfo-SMCC (sulfosuccinimidyl 4-(N-maleimidomethyl)cyclohexane-1-carboxylate) and are unusually stable up to pH 7.5 because of the cyclohexane bridge in the spacer arm.

Cross-functional linker of the invention may have the general structure reported below 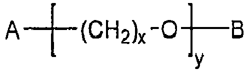 wherein A and B include different reactive groups, x is an integer from 2 to 10 (such as 2, 3 or 4), and y is an integer from 1 to 50, for example, from 2 to 30 such as from 3 to 20 or from 4 to 12. Non limitative examples of cross-linkers of this structural class are reported below.

Poly(ethylene glycol) (N-hydroxysuccinimide 5-pentanoate) ether N'-(3-maleimidopropionyl)aminoethane (Cas No. 851040-94-3; MAL-PEG-NHS ester):

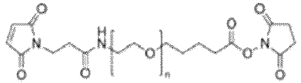

Mal-amido-PEG-TFP ester indicates a PEG linker containing a maleimide and TFP ester end group. Maleimide groups are reactive with thiols between pH 6.5 and 7.5. The TFP ester can react with primary amine groups and is also less susceptible to undergo hydrolysis compared to NHS ester. The hydrophilic PEG chains increase the water solubility of a compound in aqueous media. Longer PEG chains have improved water solubility relative to shorter PEG chains. The PEG linker has a variable number of glycol units, such as the MAL-dPEG^{®}8-TFP ester, MAL-dPEG^{®}4-TFP ester

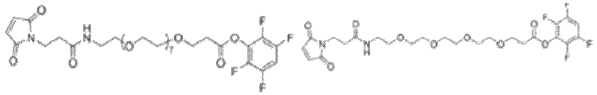

Propargyl-PEG-NHS ester is an amine reactive reagent that can be used for derivatizing peptides, antibodies, amine coated surfaces etc. The alkyne group reacts with azide-bearing compounds or biomolecules in copper catalyzed Click Chemistry reactions. It comprises for example propargyl-PEG1-NHS ester with one glycol unit and propargyl-PEG4-NHS ester which is a 4-unit amine reactive PEG linker

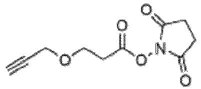

Azido-PEG-TFP ester is a click reagent containing an azide group and a TFP moiety. The azide group enables Click Chemistry. The TFP ester was used to label the primary amines (-NH2) of proteins, amine-modified oligonucleotides, and other amine-containing molecules. Chemical structure of representative compound (2,3,5,6-Tetrafluorophenyl 3-[2-[2-[2-(2-azidoethoxy)ethoxy]ethoxy]ethoxy]propanoic acid) is depicted below:

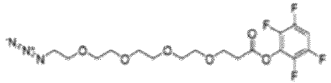

Similar PFP derivative (perfluorophenyl 1-azido-3,6,9,12-tetraoxapentadecan-15-oate; N3-PEG4-PFP) has the structure below:

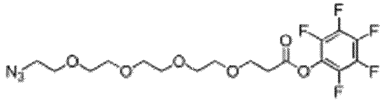

Azido-PEG-NHS ester is a PEG reagent which contain an azide group and an NHS ester. A non limitative example is the 2,5-Dioxo-1-pyrrolidinyl 3-[(23-azido-3,6,9,12,15,18,21-heptaoxatricos-1-yl)oxy] propanoate:

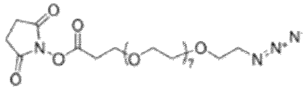

### Thiol PEG

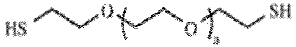

### Thiol-PEG-acid

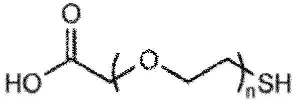

Thiol-PEG-amine

   H₂N-(CH₂CH₂O)n-CH₂CH₂-SH
Thiol-PEG-azide

   N₃-(CH₂CH₂O)ₙ-CH₂CH₂-SH
Propargyl-PEG-MAL 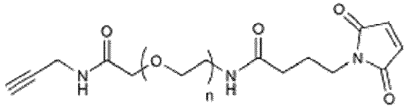
Azido-PEG-MAL

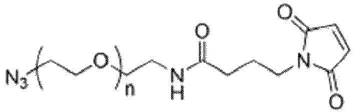

In the above compounds the polyethylene glycol chain (PEG) has a molecular weight comprised between 0.05-40 KDa, preferably lipoamide/lipoic acid-PEG-MAL and the PEG has a molecular weight of 5 KDa.

According to the present invention the metal nanoparticles coated with a polymer bearing thiol groups and -NH groups are coupled via the aforementioned heterobifunctional crosslinker to a ligand capable of recognizing a tumor-associated or inflammation-associated component.

Any of the agents of the described embodiment can be prepared in solution or can be prepared in a lyophilized form. With the term "lyophilized" is meant also dried, freeze-dried or precipitated by means of water-miscible organic solvents such as acetone.

It also object of the present invention a composition comprising the described theragnostic agents; the composition according to the invention comprises at least one of water, physiologic solution/saline, Dulbecco's Modified Eagle Medium (DMEM), Dulbecco's phosphate-buffered saline (DPBS), HEPES buffer, TRIS buffer, PIPES buffer each containing metal divalent ions such as Ca²⁺, Mg²⁺, a pharmaceutical acceptable excipient, a pH regulator.

In particular, for use in treatment, the composition can be prepared comprising, in addition to the above-described components, one or more medicament, in particular at least one of a chemotherapeutic agent, an immunomodulator, an immune cell.

In a preferred embodiment chemotherapeutic agent is selected from the group of: mitomycin-C, Bacillus Calmette Guerin (BCG), doxorubicin, melphalan, gemcitabine, taxol, cisplatin, vincristine, or vinorelbine; more preferably the immunomodulator is an anticancer vaccine and/or an immune check point blocker, such as anti-PD1 or anti-PDL1 or anti-CTLA4 antibodies, and more preferably the immune cell is a lymphocyte or a genetically modified T-lymphocyte, such as CAR-T cells, or TCR redirected T-cells or NK cells.

In a preferred embodiment the invention encompasses a composition comprising gold nanoparticles, preferably nanorods according to the invention, coated with thiolated chitosan, wherein the heterobifunctional linker is maleimido-PEG₁₂-NHS ester and the targeting moiety is an aptamer that binds to the alpha 5 subunit of integrin, preferably the Apt-Itg as herein defined.

It is also object of the present invention a kit comprising single use vials containing the multifunctional system as described above, a solvent, preferably physiologic solution/saline, Dulbecco's Modified Eagle Medium (DMEM), Dulbecco's phosphate-buffered saline (DPBS), HEPES buffer, TRIS buffer, PIPES buffer each containing metal divalent ions such as Ca²⁺, Mg²⁺, for resuspending the nanoparticles, optionally syringes and instruction for use.

It is also object of the present invention the application of the described multifunctional system, compositions and kits in the medical and diagnostic field.

In particular the nanoparticles object of the present invention with photoacoustic properties are developed for use in the diagnosis of solid tumors or inflammation and can be used in particular for the early detection of small cancer lesion, in particular urothelial, bladder gastroesophageal, colorectal, pancreatic, ovarian, lung, cervix, breast and renal cancer, brain tumors, hollow organ tumors and hepatocellular carcinoma. The nanoparticles, as disclosed by the present invention, are particularly suitable to detect urothelial and bladder cancer and actinic cystitis chronic by photoacoustic imaging.

In a preferred embodiment nanoparticles according to the present inventions can be used in the early diagnosis of bladder lesions, in particular of bladder cancer. Are therefore object of the present invention the disclosed nanoparticles for use in a method of in vivo diagnosis of bladder cancer in particular of small and flat urothelial lesions of high-grade bladder carcinoma in situ (CIS).

It is in fact possible to proceed with intravesical instillation of urine-stable nanoparticles designed according to the invention, having photoacoustic properties, handling urine containing the said nanoparticles in the bladder to avoid sedimentation and then use a multimodal imaging of the targeted lesions with PAI.

In a preferred embodiment the targeted area can be subject to thermal ablation; the delivery of the continuous light will irradiate the targeted area with the bound nanoparticles. Assisted photothermal therapy is generated by the excitation of particles at a chosen wavelength.

It is therefore object of the present invention the application of the described multifunctional system, composition and kits in the medical and diagnostic field, in particular for theragnostic application.

In a preferred embodiment the functionalized nanoparticles or compositions according to the present inventions can be used in combination with a medicament, in particular chemotherapeutic agent, a immunomodulator, an immune cell in a method of combination therapy wherein the administration of the nanoparticles and of the medicament can be simultaneous, contemporaneous or sequential.

In a preferred embodiment chemotherapeutic agent is selected from the group of: mitomycin-C, Bacillus Calmette Guerin (BCG), doxorubicin, melphalan, gemcitabine, taxol, cisplatin, vincristine, or vinorelbine; more preferably the immunomodulator is an anticancer vaccine and/or an immune check point blocker, such as anti-PD1 or anti-PDL1 or anti-CTLA4 antibodies, and more preferably the immune cell is a lymphocyte or a genetically modified T-lymphocyte, such as CAR-T cells, or TCR redirected T-cells or NK cells.

In order to demonstrate the effectiveness of the targeted multifunctional system according to the invention and in particular of nanoparticles object of the present invention the invention provides Gold nanorods (GNRs) that have been chemically engineered with a thiol-modified chitosan (Chit-SH), a bifunctional linker and an aptamer selective for the α5β1 integrin to enable tumor targeting; said particles are developed for use in a method of in vivo diagnosis and treatment of bladder cancer based on the intravesical instillation of said urine-stable targeted GNRs and, the multimodal imaging of cancer lesions with PAI.

The inventors developed a combination of strategies that allow for the detection of this tumor with an unprecedented sensibility and stability. The results show that the combination of PAI and intravesical instilled GNRs@Chit-Apt-Itg in an orthotopic model of bladder cancer can reveal the presence of lesions undetectable with US imaging and bioluminescence. The technological platform, according to the invention, could detect neoplastic lesions smaller than a half millimeter, with a sensitivity that far exceeds that of the US and CT urography for bladder carcinoma. The inventors were able to realize GNRs that can be used to detect orthotopic murine bladder cancer lesions <0.5 mm, undetectable by US imaging and bioluminescence. The inventors were able to detect up to 250 µm neoplastic lesion in the upper part of the bladder of experimental mice. According to a preferred embodiment the targeted nanoparticles of the invention are gold nanorods designed with a diameter of 10 nm and aspect ratio of 3.6 in order to have a peak light absorption at 808 nm, to leverage the optical window that allows for deeper tissue penetration and to overcome the different endogenous contrast molecules present in tissues. It was investigated and established the PA dynamic range of the above nanoparticles and identified the maximum fluence and energy of the pulsed laser light to obtain diagnostic imaging using targeted nanoparticles avoiding reshaping of the nanostructure. The gold nanoparticles according to the invention maintain more than 90% of the original light absorbance in the presence of urine in a time frame of 2 hours.

The urinary bladder environment in particular offers the possibility to exploit the intravesical instillation of the targeted nanoparticles, which is characterized by pro and cons compared to systemic delivery. Intravesical instillation allows for the avoidance of off target effects and off-target accumulations, such as the accumulation of gold in the liver, spleen, kidney, testis and brain, as has been observed in cases of systemic instillation. On the other hand, one of the problem to be solved is that intravesical delivery of the treatment i) must content with urine, which contains a broad variety of byproducts from the metabolism of endogenous and exogenous substances, bacteria, bacteria-derived mucus and floating urothelial cells, ii) is characterized by temporary retention, and iii) cannot exploit the enhanced permeability of the tumor vasculature and retention effects of the neoplastic vasculature to accumulate the intravenously injected target nanoparticles in the neoplastic environment.

Another application of the product is for urinary cytology to screen patient's urine for cancer cells. The GNRs@Chit-Apt-Itg are used for the isolation of exfoliated cancer cells present in the urine.

### MATERIALS AND METHODS

**Ethics approval.** All procedures and studies involving mice were approved by the Institutional Animal Care and Use Committee of San Raffaele Scientific Institute and performed according to the prescribed guidelines (IACUC, approval number 942).

**Tumor cell lines.** All human bladder cancer cell lines were from ATCC and cultured according instructions. RT112 cell line was from Merck (catalog number 85061106). The murine bladder cancer cell line MB49-Luc were kindly provided by Prof. Carla Molthoff (VU University Medical Center, The Netherlands) as used as recently reported¹².

**Murine orthotopic bladder tumor model.** The orthotopic bladder tumor in mice develops after the intravesical instillation of murine bioluminescent MB49-Luc bladder cancer cells, as recently reported¹². Briefly, female albino C57BL/6 J mice (9 weeks old, weighing about 20 g, Charles River Laboratories, Italy) were anesthetized with ketamine (80 mg/kg) and xylazine (15 mg/kg) and kept in dorsal position. Using a 24-gauge catheter, the bladder of each mouse was emptied and instilled with or without MB49-Luciferated (MB49-Luc) cells (10⁵ cells/100 µl in DPBS). Thirty minutes later the catheter was removed, and mice were allowed to recover and returned to their cage.

**Aptamers.** The SH-terminated 2'F-Py RNA Apt-Itg aptamer (5'-Thiol-C6-G(2'F-C)GGA(2'F-C)GGA(2'F-C)AGAGAG(2'F-U)G(2'F-C)AA(2'F-C)(2'F-C)(2'F-U)G(2'F-C)(2'F-C)G(2'F-U)G(2'F-C)(2'F-C)G(2'F-C)-3') and unrelated aptamer (5'-Thiol-C6-(2'F-U)(2'F-U)(2'F-C)G(2'F-U)A(2'F-C)(2'F-C)GGG(2'F-U)AGG(2'F-U)(2'F-U)GG(2'F-C)(2'F-U)(2'F-U)G(2'F-C)A(2'F-C)A(2'F-U)AGAA(2'F-C)G(2'F-U)G(2'F-U)(2'F-C)A-3') were purchased from Metabion (Martinsried, Germany).

**Protein extraction and immunoblot.** Cells were lysed in a buffer containing 50 mM Hepes (pH 7.5), 150 mM NaCl, 1% glycerol, 1% Triton X-100, 1.5 mM MgCl2, 5 mM EGTA, supplemented with 1 mM Na3VO4 and protease inhibitors (Roche Diagnostics, Indianapolis, USA). After incubation on ice for 20 min, the extracts were clarified by centrifugation at 13,200 rpm for 10 min at 4°C. Protein concentration was determined by the Bradford colorimetric assay (Bio-Rad, Hercules, CA) using bovine serum albumin as the standard. SDS-PAGE was carried out according to Laemmli. Gels were electroblotted into polyvinylidene difluoride membranes (Amersham BioSciences, UK) and filters were probed with anti-Integrin beta 1 (clone EPR16895, Abcam, Cambridge, UK), anti-Integrin alpha 5 (clone EPR7854, Abcam) or anti-α-tubulin (DM1A, Cell Signaling Technology, Danvers, MA, USA) primary antibodies, followed by the HRP-conjugated secondary antibodies. Densitometric analysis was performed on at least two different expositions to assure the linearity of each acquisition using ImageJ (v 1. 46r).

**Integrin α5 silencing.** Human T24 and RT112, and murine MB49-Luc cells (1.8 × 10⁵) were seeded in 6-well plates and after 24 hours were overlaid with the transfection mixtures containing 60 nM small interfering RNAs (siRNAs) targeting murine and human integrin α5, respectively, and Lipofectamine RNAiMAX Reagent (ThermoFisher Scientific) in Opti-MEM I reduced serum medium (Invitrogen), according to the manufacturer's instructions of transfection reagent. siRNAs (Qiagen, Hilden, Germany) tested were: HS_ITGA5_7, HS_ITGA5_5, HS_ITGA5_4 and HS_ITGA5_2 (T24 cells); HS_ITGA5_7 and HS_ITGA5_5 (RT112 cells); MM_ITGA5_1, MM_ITGA5_2, MM ITGAS3 and MM_ITGA5_4 (MB49-Luc cells). Scrambled (SCR) non-targeting siRNA (siRNA ctrl, Qiagen, Hilden, Germany) was used as a negative control. After 5-hour incubation, complete culture medium was added to the cells and incubation was prolonged up to 24 hours. Silencing efficiency was assessed by immunoblotting.

**Cell Binding Assay.** Binding of Apt-Itg to human T24 and RT112, and murine MB49-Luc cells was assessed by flow cytometry. Apt-Itg and unrelated aptamer were internally labelled with Alexa Fluor 647 fluorescent probe by using the Ulysis Nucleic Acid Labeling Kit (Invitrogen), which provides a non-enzymatic method for chemically labeling purine bases in the entire sequence with the fluorescent dye.

Cells were detached from culture plates with 0.02% EDTA and washed twice with 500 µl Dulbecco's phosphate-buffered saline (DPBS). Cells (2.0 × 10⁵) were left untreated or incubated with increasing concentrations of Alexa Fluor 647-Apt-Itg or unrelated aptamer in binding buffer (DPBS supplemented with 1 mg/ml yeast tRNA and 1 mg/ml ultrapure salmon sperm DNA, as nonspecific competitors), with or without 1 mM MgCl₂ and 0.5 mM CaCl₂. After 10 min at room temperature (RT), cells were washed three times with 100 µL DPBS, suspended in 100 µl DPBS and analysed by flow cytometry (BD Accuri^{™} C6). Data analysis was performed using FlowJo software (version 10.0.7). The geometric mean fluorescence intensity (gMFI) of aptamer-treated samples was normalized to the gMFI of the cells alone and the apparent dissociation constant (Kd) of the aptamer-cell interaction was obtained by fitting the dependence of relative gMFI on the concentration of aptamer to the one-site saturation equation Y = Bmax × X/(Kd + X), using GraphPad Prism version 6.00, as previously described^{18,19}. Binding curves represent data from at least two independent experiments.

Binding of 1 µM Alexa 647-Apt-Itg to human and murine bladder cancer cell lines transfected with HS_ITGA5_7 and MM_ITGA5_4 (both referred to as si-ITGα5), respectively, was performed after 24 hours from transfection using flow cytometry, as described above.

**Intravesical instillation of GNRs@chit-Apt-Itg.** GNRs@Chit-Apt-Itg were produced and instilled into the bladder through a catheter, as reported¹². The only reported protocol modification is that, instead of using ultrasound-mediated shaking, GNRs instilled into the bladder lumen were kept in suspension by flushing the bladder 3 times through a catheter that was maintained in the urethra for 15 min, a procedure that is in agreement with the current operations made in the surgery room where the surgeon flushes the bladder with saline solution.

**In vivo ultrasound (US) and photoacoustic imaging (PAI) of mice instilled with GNRs@Chit-Apt-Itg.** Photoacoustic (PA) and ultrasound (US) imaging was carried out on female albino C57BL/6 J mice bearing orthotopic bladder tumour following intravesical instillation with GNRs@Chit-Apt-Itg. High-resolution US and PA imaging have been acquired using the Vevo LAZR-X platform (FUTIFILM VisualSonics, Inc.,Toronto, ON, Canada). The imaging platform includes a high frequency US system (Vevo 3100) combined with an Nd:YAG nano-second pulsed laser with repetition rate of 20 Hz. The linear US transducer array Mx 550D consists of 256 elements with a nominal center frequency of 40 MHz (25-55 MHz bandwidth) and a spatial resolution of 40 µm with a maximum imaging depth of 15 mm. Light from the laser is delivered to the tissue through optical fibers mounted on either side of the transducer; since the GNRs are susceptible to change in shape at the higher laser threshold, light attenuators were prepared to reduce the laser fluence to avoid GNRs reshaping, as recently reported¹². During volumetric US-PA acquisitions, a stepper motor is used for the linear translation of the US transducer and optical fibers along the sample. The linear stepper motor moves in steps of a minimum of 0: 1 mm while capturing 2-D parallel images, for a maximum 3D range distance of 6.4 cm.

3D B mode scan was carried out for mouse bladder. The photoacoustic spectra between 680 nm and 970 nm were scanned with a step size of 5 nm; for the in vivo studies (murine bladder) the 3D multispectral PA scans were acquired selecting PA spectral curve of tissue components melanin, deoxy- and oxy-genated blood, and GNRs; the processed wavelengths (680; 722; 764; 810; 924; 970 nm) were automatically selected from the spectral curve used to spectrally unmix the GNRs signal from other endogenous tissue chromophore signals such as oxy, deoxy hemoglobin. The algorithm reported by Luke et al.²⁰ was used to select these wavelengths which is ideal for separating the signal from GNRs from other endogenous absorbers. Data analyses were conducted using the Vevo^{®}Lab software; volumes of interest were obtained by manually drawing Volumes of Interest (VOIs) on 3D B-mode images. GNRs, melanin, oxy- and deoxy-hemoglobin content were estimated through spectral unmixing analyses of the spectroscopic data.

***In vitro* PAI of GNRs.** The PA spectra of GNRs@Chit and GNRs@Cit-Apt-Itg was evaluated in vitro, using agar drop, as recently reported¹². Briefly, GNRs (30 µl in DPBS with calcium and magnesium) were mixed with a 1% agar solution (30 µl), the mixture was then poured on Parafilm^{®} M (Sigma) and left to solidify in a humidified chamber. The solidified product (called "agar drop") was then placed on an ultrasound gel pad (Aquaflex, Parker), embedded in slime made of polyvinyl alcohol polymer crosslinked with sodium tetraborate (Barrel of slime) and PAI was performed using the two optical fiber bearing light attenuators. The transducer was perpendicularly placed on the object under investigation previously covered with ultrasound transmission gel (Aquasonic 100, Parker). Axial sections were acquired using the following settings for B-Mode: 2D Power 100%; 2D Gain 13 dB; for PA-mode: Pa Power 100%; PA gain *44 dB*; TGC and depth were maintained identical for all drops. PA and US data have been analyzed using VevoLab 3.2.5 software.

**Synthesis of GNRs@CTAB** with maximal absorption at 800 nm. Cetyltrimethylammonium bromide (CTAB)-coated GNRs (GNRs@CTAB) with a maximum absorption of 800 nm, were prepared according to a previously published procedure with minor modifications [1] and scaled to a volume of 2 liters. The initial solution for the growth of GNRs was prepared by dissolving CTAB (31.14 g, 85.4 mmol) and sodium oleate (4.28 g, 14.0 mmol) in 1.8 L of warm water (~ 50°C) in a thermostatically controlled 2-L jacketed reactor, equipped with mechanical stirring. When the solution reached 30°C, 810 µL of AgNO₃ solution (0.4 M in ultrapure water) were added and the mixture was then incubated for 15 min without stirring. Then, under continuous stirring (700 rpm), 8.652 mL of HAuCl₄ solution (0.1 M in ultrapure water) were added. Au(III) was reduced to Au(I) thanks to sodium oleate for 90 min, after which 3.56 mL of hydrochloric acid (37 %) and 3.6 mL of ascorbic acid (0.079 M) were added to adjust the pH to 1.0 and to ensure the complete reduction of the gold precursor. Separately, a seed solution was prepared by dissolving 364 mg (1.0 mmol) of CTAB in 10 mL of warm water in a 50 mL round-bottomed flask. After cooling down to room temperature 25 µL of HAuCl₄ solution (0.1 M) were added while stirring. The seeds were formed by quickly injecting 600 µL of ice-cold sodium borohydride (0.01 M) into the solution, causing the color of the solution to change from yellow to brown, indicating the formation of ultra-small gold seeds. Finally, after ageing the seed solution for 30 minutes at room temperature, 690 µL of it were added to the growth solution, which was vigorously stirred for 30 s then left undisturbed overnight at 30°C to allow for GNRs growth. Purification of GNRs@CTAB was performed by i) centrifugation in 50-mL Falcon tubes (6000 rpm for 100 min), ii) removal of 40 mL of the supernatant and iii) redispersion in 40 mL of ultrapure water; the process was repeated 3 times. The final product was then collected in 200 ml of ultrapure water. The gold concentration in GNRs@CTAB was 2.09 mM, as determined by atomic absorption spectroscopy.

GNRs were designed for displaying peak absorption in the NIR biological window, placed around 800 nm. The synthesis of GNRs@CTAB performed in a 2-L jacketed reactor is able to produce 200 mL of 2.09 mM GNRs@CTAB (Yield = 49%), determined by Flame Atomic Absorption Spectroscopy (FAAS), and a total dry matter of 0.8 mg/mL, measured by gravimetric analysis. This means that the gold content is approximately 60% of the nanosystem mass, in agreement with the fact that the CTAB completely covers the gold core and an excess of surfactant in solution is required to keep the nanosystem stable. The surface zeta potential is + 29.8 mV, as expected for the model of CTAB-capped gold nanosystems.

**Coating of GNRs with chitosan (GNRs@Chit).** For this system, chitosan was used for coating the nanostructures: this biopolymer is nowadays extensively used in biomedical applications thanks to its biocompatibility and biodegradability; chitosan is also employed in the field of nanoparticles due to its capacity to stabilize GNRs during the synthesis process, not including any toxic reagent. Moreover, chitosan is full of NH₂ groups that allows the functionalization with amine-reactive linkers in order to perform the conjugation with several drugs, antibodies or peptides. GNRs@CTAB were covered with chitosan by means of a thiolated-chitosan conjugate. This thiol insertion happens on the single chitosan monomer and is performed through carbodiimide-assisted coupling between amino groups of chitosan and the carboxylic acid moiety of thioglycolic acid. The effective modification of chitosan with thiol groups was confirmed by the mean of 1H-NMR.

Medical grade chitosan (500 mg, 3.1 mmol) was dissolved in 50 mL of 1 vol. % acetic acid and mixed with 0.5 mL (7.2 mmol) of thioglycolic acid under moderate stirring. N-(3-dimethylaminopropyl)-N'-ethylcarbodiimide hydrochloride (500 mg, 2.6 mmol) was then added to activate the carboxylic group of thioglycolic acid and to promote the coupling to the amino groups of chitosan. The reaction was left to incubate for 6 h at room temperature under continuous stirring. The product was then dialyzed using a 3.5 kDa cut-off dialysis tube overnight against ultrapure water and the resulting thiolated-chitosan was diluted to 500 mL with water. At this point, 30 mL of GNRs@CTAB were added dropwise under mild stirring and the resulting solution was incubated (48 h, room temperature) to allow for the coupling of thiolated-chitosan and GNRs. The product was subsequently concentrated using an Amicon^{®} stirred cell equipped with PES membranes (100 kDa cut-off, using 4 bar nitrogen pressure) to remove CTAB, and the final product (60 mL), called GNRs@Chit, was then split in aliquots, freeze dryed and stored at +4 °C until the subsequent step.

The replacement of CTAB with chitosan first and the binding does not modify the shape, morphology, or optical properties of the GNRs, as they result perfectly preserved. The whole replacement of CTAB was assessed by 1H-NMR and by mean of surface zeta potential measurement of purified GNRs that ranged from + 35 mV to + 45 mV. This increased zeta potential suggests that the exchange CTAB/Chit happened, being that the residual non-functionalized amino groups of chitosan preserve their cationic nature in water.

**Functionalization of GNRs@Chit with aptamers.** GNRs@Chit was functionalized with Apt-Itg and unrelated aptamer. 10 ml of Maleimide-PEG12-NHS (1 mg/ml in ultrapure water, 11.55 µmol = 10 mg) were mixed with 10 ml of GNR@Chit (1 mM of Au, 10 µmol = 1.969 mg Au) under stirring to achieve a final weight ratio of Maleimide-PEG12-NHS:Au = 5: 1. The mixture was then left to incubate overnight at RT and then dialyzed against ultrapure water using a 3.5 kDa cut-off dialysis tube for 24 h, at RT, to remove the excess of the crosslinker. The product (25 ml), consisting of activated GNRs@Chit-PEG12-maleimide, was then mixed with pre-established appropriate amount of aptamer (Apt-Itg or Unrelated), previously dissolved in 1 ml of water and activated by three cycles at 85°-0°-37°C. The mixture was left to react for 24 h. The day after, the solutions of GNRs@Chit-Apt-Itg were washed and concentrated by using Slide-A-Lyzer Dialysis Cassettes (3.5 kDa MWCO), freeze dried in sterile conditions and stored at +4 °C until the shipping. Each vial, at the end of the fabrication, contains 98.48 µg Au and has to be redissolved in 500 µL of water or saline for reaching 1 mM [Au] final concentration. The dry content for the freeze-dried vial is about 4.2 mg each.

The inventors exploited a heterobifunctional crosslinker reagent, composed of an ethylene oxide spacer (PEG12) bearing a N-hydroxysuccinimidyl (NHS) ester to its extremities and a maleimide functional group (NHS-PEG12-maleimide). The NHS ester terminus reacts with the free amino groups on chitosan ensuring the binding of the linker to the chitosan, while the maleimide group is available to react in a further step with the sulfhydryl group of the aptamer, expressly designed with the thiol at the 5' end. Apt-Itg or Unrelated was then coupled with activated GNRs@Chit, prior a short denaturation-renaturation step (85 °C for 5 minutes, snap-cooled on ice for 2 minutes, and allowed to warm up to 37 °C).

**Quantification of the Apt-Itg bound to the GNRs@Chit-Apt-Itg.** To determine the amount of aptamers bound to GNRs@Chit-Apt-Itg, 5 µl of nanoparticles were incubated with aptamer specific 3' primers, heated at 65°C for 5 min and annealed at 22°C for 5 min. RNA was reverse transcribed (RT) using Tetro Reverse Transcriptase (Bioline, London, UK) at 42°C for 15 min followed by an extension at 50°C for 30 min and enzyme inactivation at 95°C for 5 min. The product from the RT reaction was PCR amplified with iQ SYBR Green Supermix (Bio-Rad, Hercules, CA) in the presence of the Apt-Itg or unrelated aptamer 5' and 3' primers. The qPCR protocol was as follows: the RT product was heated at 95°C for 2 min, followed by 40 cycles of heating at 95°C for 30 sec, annealing at 55°C for 30 sec, and extending at 60°C for 30 sec. A melt curve stage by heating at 60-95°C was performed. Reactions were all done in 25 µL volume in triplicate.

The primers used were:
Apt-Itg: 5' primer, 5'-AGCGGACGGACAGAG-3' (SEQ ID No: 3); 3' primer, 5'-GCGGCACGGCAGGTT-3' (SEQ ID No: 4).
Unrelated aptamer: 5' primer, 5'-TAATACGACTCACTATAGGGTTCGTACCGGGT-3' (SEQ ID No: 5); 3' primer, 5'- TGACACGTTCTATGTGCA-3' (SEQ. ID No: 6).
The quantity of the amplified product was extrapolated from a standard amplification curve obtained with unconjugated aptamers. Three independent experiments were performed.
**Analysis of GNRs@Chit-Apt-Itg stability in human urine.** Half mL of water was added to a lyophilized vial of functionalized gold-nanorods (GNRs@Chit-Apt-Itg), obtaining a concentration of 1 mM of gold.
0.1 mL of GNRs@Chit-Apt-Itg were added to 3 mL of human urine from a healthy volunteer and subjected to VIS-NIR spectroscopy (Cary 3500 UV-VIS-NIR modular spectrometer, Agilent Technologies, Santa Clara, USA) using a 1 cm path-length plastic cuvette, collecting absorbance values between 500 and 1100 nm at various time points at 25°C. Undiluted human urine solution was used as reference for the analysis.

### Results.

**Aptamers.** New 34-mer Apt-Itg aptamer was derived from the reported 68-mer H02 unmodified RNA sequence¹⁵ by removing nucleotides from 1 to 17 and 52 to 68 in the fixed flanks of the full-length molecule. The secondary structure and free energy change (ΔG) of the new and shorter aptamer (Apt-Itg) was predicted by using Mfold web server²¹ (Figure 1); the ΔG value of -14.4 Kcal/mol indicates the high structural stability of the Apt-Itg.

In the present invention, all of the pyrimidine residues in Apt-Itg sequence are modified to 2'F-Py to enhance RNA stability.

The sequence of the 2'F-Py RNA Apt-Itg is the following:

5'-G(2'F-C)GGA(2'F-C)GGA(2'F-C)AGAGAG(2'F-U)G(2’F-C)AA(2’F-C)(2’F-C)(2'F-U)G(2’F-
C)(2’F-C)G(2'F-U)G(2’F-C)(2’F-C)G(2’F-C)-3' (SEQ ID No:2)

In the invention, the sequence of the 2'F-Py RNA Apt-Itg is modified at the 5'-end by adding a Thiol-C6 group for functionalization of GNRs@Chit.

The sequence of the 2'F-Py RNA Apt-Itg modified with Thiol-C6 group is the following:

5'-Thiol-C6-G(2'F-C)GGA(2'F-C)GGA(2'F-C)AGAGAG(2'F-U)G(2’F-C)AA(2’F-C)(2’F-C)(2'F-
U)G(2’F-C)(2’F-C)G(2'F-U)G(2’F-C)(2’F-C)G(2’F-C)-3' (SEQ ID No: 7)

The SH-terminated 2'F-Py RNA with no affinity for integrin α5β1, consisting in the scrambled sequence of a previously generated anti-EGFR aptamer²² was here used as negative control, and is indicated as "Unrelated".

The sequence of the 2'F-Py RNA unrelated aptamer is the following:

5’-(2'F-U)(2'F-U)(2'F-C)G(2'F-U)A(2'F-C)(2'F-C)GGG(2'F-U)AGG(2'F-U)(2'F-U)GG(2'F-C)(2'F-
U)(2'F-U)G(2'F-C)A(2'F-C)A(2'F-U)AGAA(2'F-C)G(2'F-U)G(2'F-U)(2'F-C)A-3' (SEQ ID No: 8).

The sequence of the 2'F-Py RNA unrelated aptamer modified with Thiol-C6 group is the following:

5'-Thiol-C6-(2'F-U)(2'F-U)(2'F-C)G(2'F-U)A(2'F-C)(2'F-C)GGG(2'F-U)AGG(2'F-U)(2'F-
U)GG(2'F-C)(2'F-U)(2'F-U)G(2'F-C)A(2'F-C)A(2'F-U)AGAA(2'F-C)G(2'F-U)G(2'F-U)(2'F-C)A-3'
(SEQ. ID. No:9).

In order to confer active targeting abilities to GNRs@Chit toward integrin α5β1-positive MB49 cells, the nuclease-resistant 2'F-Py RNA Apt-Itg aptamer was used as the targeting agent.

Apt-Itg has the following advantages over the original 68-mer H02 longer sequence: 1) easier chemical synthesis at lower costs and higher yield; 2) easier conjugation to GNRs@Chit nanosystem; 3) increased resistance toward nucleases due to the modified pyrimidine residues²³.

**Identification of the integrin α5β1 on bladder cancer cell lines.** Murine and human bladder cancer cell lines were searched for the expression of the integrin α5β1. Recently published data showing that human bladder cancer cell lines express the integrin α5β1¹² were confirmed, and here it was further demonstrated that the integrin α5β1 is mainly expressed by bladder cancer cells of grade 2/3 (RT4=G1; T24=G3; 5637=G2; HT-1376=G3; RT112=G2), as the human in situ carcinoma that always appears as high grade/grade 3³. The murine bladder cancer cell line MB49-Luc also expressed the integrin α5β1, similarly to the human bladder cancer cell line of grade 2/3 (Figure 2).

**Apt-Itg binds to human and murine bladder cancer cell lines with nanomolar affinity.** Since H02 was selected to bind to the human integrin α5¹⁵, the inventors first checked whether Apt-Itg contains the H02 active site and binds to human integrin α5β1-positive T24 and RT112 bladder cancer cell lines, expressing high and moderate levels of integrin α5, respectively, and comparable levels of integrin β1 (Figure 2). Binding was assessed through flow cytometric assays using internally Alexa 647-labeled aptamer. As shown (Figure 3), Apt-Itg binds to T24 cells with a Kd value of 222.2 ± 38.1 nM which is somewhat comparable to that observed for the full-length H02 aptamer on human glioblastoma U87MG cells genetically modified to overexpress integrin α5 (277.8 ± 51.8 nM). A higher Kd value (450.6 ± 95.6 nM) was calculated on RT112 cells, thus reflecting the lower expression level of α5 on these cells compared to T24 cells. By contrast, the unrelated control aptamer gave no appreciable binding on the two cell lines, even at concentrations as high as 4 µM.

Next, in order to test GNRs@Chit conjugated to Apt-Itg in mice bearing MB49-Luc-derived tumors, the capability of Apt-Itg to bind to murine MB49-Luc cells was checked. Even if some species specificity has been shown in aptamers recognition ability²⁴, inventors hypothesized a high affinity binding for Apt-Itg to murine integrin α5, which shares about 90% sequence identity, according to BLAST analysis, in the extracellular region with the human receptor. As expected, Apt-Itg efficiently binds to MB49-Luc cells, with a Kd value of 247.5 ± 72.5 nM, even in the presence of pre-treatment of cells with a 30-fold excess of unrelated aptamer that shows almost undetectable nonspecific binding on cells (Figure 4).

**Apt-Itg binds human and murine cancer cells also in the presence of divalent cations.** Integrins contain binding sites for divalent cations (such as calcium and magnesium) that regulate their ligand-binding affinity. We tested the binding capacity of the aptamenr that recognized the a5b1 integrin, and we report no significant changes in Kd values of Apt-Itg, on either human and murine cancer cell lines in the presence of physiological concentrations of Ca²⁺ and Mg²⁺ cations (Figure 5). These data show that the integrin α5 recognition by the aptamer is not affected by cation-triggered conformational changes of the receptor.

**Silencing of the integrin α5 reduced the binding of the Apt-Itg.** In order to confirm the specificity of Apt-Itg aptamer for integrin α5, siRNA was used to reduce integrin α5 expression in human T24 and RT112 cells, and murine MB49-Luc. Compared with siRNA ctrl, the tested anti-α5 siRNAs specifically reduced the expression of α5 (Figure 6). Importantly, Apt-Itg binding to either human and murine cells transfected with HS_ITGA5_7 and MM_ ITGA5_4, respectively, was significantly reduced compared to binding to siRNA ctrl treated cells (Figure 7). Overall, these results indicate Apt-Itg as a viable integrin α5-targeting candidate for nanovectors functionalization.

**Quantification of Apt-Itg bound to GNRs@Chit by Real-time PCR.** To evaluate the efficiency and reproducibility of the conjugation of the Apt-Itg to the nanoparticles, GNRs@Chit were left reacting with three different amount of Apt-Itg, such as 100, 50 and 10 pmol. The resulting product was called GNRs@Chit-Apt-Itg "TOP", 1/2 sample and 1/10 sample. For the unrelated aptamer only the 'TOP' concentration was tested. From the three preparation of GNRs@Chit-Apt-Itg the inventors quantified by RT-qPCR analysis 4.0, 1.9 and 0.7 pmol of Apt-Itg-SH aptamer for the 'TOP', 1/2 and 1/10 preparation, respectively (Figure 8). This data shows that the recovery of the bound Apt-Itg was 4% for the TOP and 1/2 batches of nanoparticles, and 7% for the 1/10 preparation. For the "TOP" preparation a conjugation of 1.14 pmol Itg-SH per mg of dry content was estimated, which means 45 ng of aptamer for 0.1 mg of gold. Overall, the efficiency of conjugation of the Apt-Itg ranged between 4% and 7% with a mean±SEM equal to 5±1%.

Overall, this information shows that the use of Apt-Itg allows a reproducible preparation of targeted GNRs, with low batch-to-batch variation.

**The conjugation of the Itg-aptamer does not modify the chemical-physical properties of the GNRs@Chit.** A heterobifunctional crosslinker reagent was exploited, composed of an ethylene oxide spacer (PEG12) bearing a N-hydroxysuccinimidyl (NHS) ester to its extremities and a maleimide functional group (NHS-PEG12-maleimide). The NHS ester terminus reacts with the free amino groups on chitosan ensuring the binding of the linker to the chitosan, while the maleimide group is available to react in a further step with the sulfhydryl group of the aptamer, expressly designed with the thiol at the 5' end (Figure 9A).

Transmission electron microscopy (TEM) confirmed effective synthesis of monocrystalline GNRs with the appropriate aspect ratio (3.43 ± 0.52) showing the presence of cylindrical gold nanostructures with width (25.7 ± 2.0 nm) and length (88.2 ± 6.4 nm), compatible with the NIR optical behavior of the nanosystem (Figure 9B).

Next, the physical-chemical properties of the metal core of GNRs@Chit-Apt-Itg/Unrelated were assessed, determining the longitudinal-LSPR to peak at 802 nm, shape and dispersion, and aspect ratio of 3.62 ± 0.67 (length 90.2 ± 7.2 nm, width 24.9 ± 2.6 nm. These findings show that conjugation of aptamers does not modify the physical-chemical properties of GNRs, with only small and gradual variation of the longitudinal-LSPR peak vs GNRs@Chit (800 nm) and GNR@CTAB (798 nm) that is representative of the variation of the chemical environment surrounding the GNRs (Figure 9C). Flame Atomic Absorption Spectroscopy (FAAS) and thermogravimetric analysis (TGA) were then used to establish the relative amount of gold and chitosan in the final nanosystem (Figure 9C). TGA estimated a total dry matter of 8.4 mg/mL. FAAS estimated a gold amount of 0.2 mg/mL of gold. The rheology in terms of viscosity of the solution was estimated by varying the temperature from +4°C to +40°C: the result is a constant decrease of the viscosity with increasing temperature. These findings indicate that GNRs@Chit were made of 2% Au and 98% chitosan, and that this solution is so suitable for intravesical instillation.

Both the Apt-Itg and the unrelated-conjugated nanosystems displayed a reduction in the Z-potential (+ 10.8 mV and + 10.3 mV respectively, vs. + 40.0 mV measured for GNRs@Chit), according to the reduced amount of free amino groups on chitosan due to the presence of the PEG linker. Moreover, the crystallinity and the composition of the GNRs@Chit-Apt-Itg/Unrelated was proven to be conserved after the consecutive conjugation steps, additionally revealing no detectable Br atoms from CTAB. GNRs@Chit-Apt-Itg/Unrelated was therefore aliquoted in vials, freeze-dried and vacuum sealed to achieve sterile single use vials containing the lyophilized product.

The freeze-dried GNRs@Chit-Apt-Itg/ Unrelated were then dissolved in 500 µl of 1% acetic acid in water to obtain 1 mM solution of gold. The PA properties of the GNRs were investigated using agar drops containing GNRs@Chit-Apt-Itg or GNRs@Chit. The results show that the PA spectra of GNRs@Chit-Apt-Itg is superimposable to that of GNRs@Chit, thus demonstrating that the functionalization of GNRs@Chit with the Apt-Itg does not modify the PA spectra of the GNRs@Chit (Figure 9D).

**GNRs@Chit-Apt-Itg is stable in human urine.** Urine represents a harsh environment, characterized by a complex mixture of metabolites and bacteria that could interact with the nanoparticles. Therefore, we estimated the potential impact of urine on the light absorption of the three formulations of GNRs (GNRs@CTAB, GNRs@Chit, GNRs@Chit-Apt-Itg), and the stability of the GNRs@Chit-Apt-Itg in urine in a time frame of 2 hours.

The longitudinal LSPR peak of GNRs@Chit-Apt-Itg was observed at 834 nm, consistently with the LSPR peak of the unconjugated GNRs@Chit that was at 836 nm (Figure 10A, Table 1).

The urine stability of GNRs@Chit-Apt-Itg over time was also investigated, showing only a 7% decrease of light absorbance after 2 hours (Figure 10B, Table 2).

**Table 1: Wavelength and absorbance values for GNRs with different functionalization.**

| **GNRs formulation** | **λₘₐₓ (nm)** | **Aₘₐₓ** | **A₈₀₀ₙₘ** |
|---|---|---|---|
| GNRs@CTAB | 818 | 0,573 | 0,534 |
| GNRs@Chit | 836 | 0,493 | 0,377 |
| GNRs@Chit-Apt-Itg | 834 | 0,418 | 0,336 |

**Table 2: Wavelength and absorbance values of GNRs@Chit-Apt-Itg in human urine over time.**

| **Time (min)** | **A max** | **λ (nm)** | **A%** |
|---|---|---|---|
| 0 | 0,391 | 851 | 100% |
| 5 | 0,386 | 853 | 99% |
| 10 | 0,386 | 850 | 99% |
| 15 | 0,378 | 852 | 97% |
| 30 | 0,375 | 854 | 96% |
| 45 | 0,376 | 854 | 96% |
| 60 | 0,376 | 849 | 96% |
| 120 | 0,363 | 854 | 93% |

**Feasibility and specificity of the use of GNRs@chit-Apt-Itg for the recognition of the orthotopic bladder cancer.** Before the intravesical instillation of targeted GNRs the periphery of the tumor mass was negative for the spectra of GNRs, but positive for the PA spectra of oxygenated blood (Figure 11A). After the intravesical instillation of the targeted GNRs the PA spectra of GNRs@chit-Apt-Itg was located only on the surface of the tumor mass but not on the non-neoplastic urothelium (Figure 11B). The same protocol was used for control animals in the absence of tumor, showing vascularization of the bladder wall (PA spectra of oxygenated blood) before (Figure 11C) and after the intravesical instillation of GNRs@chit-Apt-Itg (Figure 11D). These information show that GNRs@chit-Apt-Itg binds only the tumor cells, but not the non-neoplastic tissue in tumor environment nor the normal urothelium.

**The photoacoustic signal of GNRs@chit-Apt-Itg identifies tumor areas that develops in the following day.** It was next assessed whether the tissue areas identified through the PA signal of the GNRs@Chit-Apt-Itg were predictive of tumor growth, using animals with detectable tumor (Figure 12A). In the tumor bearing animals the GNRs@Chit-Apt-Itg correctly identified the tumor mass (red ROI in Figure 10B) and the surrounding tumor margins (blue and orange ROI in Figure 12B). In the following 18 hours the PA signal was lost, but the tumor was growth on the lateral sides of the mass identified the day before (Figure 12C).

**Lowering the amount of Apt-Itg loaded onto the GNRs@Chit does not impact on the recognition of the tumor mass.** It was next evaluated the effectiveness of tumor recognition using nanoparticles loaded with 1/10 of Apt-Itg previously used in Figure 9 and 10. Using the same amount of gold but loaded with 1/10 of Apt-Itg the tumor mass, but not the surrounding non-neoplastic urothelium, was identified (Figure 13). This data owns twofold information, such as that i) lowering the amount of Apt-Itg loaded onto the GNRs@Chit does not impact on the efficacy to recognize the tumor, and ii) the sites of the linker that have not been conjugated with the Apt-Itg do not deliver an unspecific binding, thus do not provide an unspecific PA signal of the GNRs.

**GNRs@Chit-Apt-Itg are better monodispersed that GNRs@Chit-Iso4.** After having established the efficacy and specificity of the GNRs@Chit-Apt-Itg to recognize the tumor mass, but not the surrounding non-neoplastic urothelium, the properties of GNRs@Chit-Apt-Itg were compared to GNRs@Chit-Iso4.

The magnitude of the zeta potential is an indication of the potential stability of the colloidal system, as particles with large negative or positive zeta potential tend to repel each other and there will be no tendency for the particles to come together²⁵. A more positive Z-potential was observed for GNRs@Chit-Apt-Itg (both, loaded with top or 1/10 amount of Apt-Itg) vs GNRs@Chit-Iso4, suggesting better colloidal property. In agreement, the polydispersity index (PdI, used to describe the degree of "non-uniformity" of a distribution) was lower for GNRs@Chit-Apt-Itg (both, loaded with top or 1/10 amount of Apt-Itg) (Table 3).

**Table 3. Analysis of the potential and of the hydrodynamic range of GNRs.**

| | Z potential (mV) | Electrophoretic mobility (mmcm/Vs) | Z-average (nm) | PdI |
|---|---|---|---|---|
| GNRs@Chit-Apt-Itg Top | +32.5 | +2.54 | 881.2 | 0.489 |
| GNRs^{®}Chit-Apt-Itg 1/10 | +33.9 | +2.65 | 822.9 | 0.462 |
| GNRs@Chit-Iso4 | +11.1 | +0,87 | 150.7 | 0.709 |

| | | | | |
|---|---|---|---|---|
| PdI; polydispersity index. Electrophoretic light scattering and dynamic light scattering were performed by an accredited laboratory, according to ISO-13099-2:2012 and ISO 22412:2017. | | | | |

Likewise, also the histogram representation showed more uniform distribution of the size of the GNRs@Chit-Apt-Itg vs GNRs@Chit-Iso4 that was composed by several clusters of size distribution (Figure 14). Also, the use of the GNRs@Chit-Apt-Itg in vivo did not require the use of ultrasound-assisted shaking to keep the nanoparticles in suspension, as indeed used for the GNRs@Chit-Iso4¹². **Racemization of the peptide Iso4.** Racemization refers to the overall loss of chiral integrity of the amino acid or peptide and involves the base-catalyzed conversion of one enantiomer (usually the L-form) of an amino acid into a mixture of L- and D-enantiomers. Indeed, racemization is a process that occurs with cyclic peptides, in particular when they contain the aminoacid phenylglycine (Phg)²⁶, as is the case of the cyclic peptide Iso4 ((head-to-tail cyclized c(Cphg*iso*DGRG)¹². Indeed, we have recently reported that the peptide Iso4 undergoes racemization of the phenilglycine²⁷, with the D and L isoforms accounting for relative abundance that is batch dependent (Figure 15A). The racemization also occurs when the Iso4 peptide was synthesized as L-Iso4 (Figure 15B).

### DISCUSSION

The present invention provides an aptamer instead of the Iso4 peptide for the recognition of tumor expressing the integrin α5β1. A new 34-mer aptamer against the integrin α5β1 (Apt-Itg) was delivered, which was stable and active in the urinary environment. The urine-stable Atp-Itg was further modified at the 5'-end by adding a Thiol-C6 group to allow for efficient binding to and functionalization of GNRs@Chit. Indeed, dose-dependent amount of Apt-Itg conjugated to the GNRs@Chit was obtained when three different concentrations of ligand were used during the process of synthesis of the GNRs@Chit-Apt-Itg. Using three different concentrations of Apt-Itg during the synthesis process, the invention demonstrated an average conjugation efficiency of 5%, in agreement with previous report²⁸, and a reduced batch-to-batch variation.

GNRs functionalized with an aptamer for the application in the urinary environment have never been reported. The nanosystem GNRs@Chit-Apt-Itg represents a novel contrast agent for the detection of α5β1+ bladder cancer with high specificity. However, the GNRs employed here can also be used for the application of photothermal therapy (PTT)^{29,30}, which is obtained by using continuous laser at the same wavelength used for the photoacoustic imaging (pulsed laser) that is 810 nm and is approved for clinical practice by the American National *Standards* Institute³¹. The nanosystem GNRs@Chit-Apt-Itg offers unmet theranostic opportunities, and being unrelated to cellular metabolism, it will overcome bias in the gender medicine³², and will be equally effective in male and female bladder cancer patients^{33,34}.

The feasibility of using intravesical instillation of nano- and micro-particles for the photoacoustic imaging of bladder cancer has been previously demonstrated, such as in our recent report that used GNRs@Chit-Iso4¹² and silicon dioxide microparticle³⁵, respectively. Indeed, both systems are biased by settling down to the bottom side of the bladder, and thus requires solutions (US-assisted shaking or magnetic field) applied to the external part of the body to keep them in suspension in the bladder. By delivering nanoparticles that have positive charge and uniform size distribution the nanosystem GNRs@Chit-Apt-Itg has overcome the above bias, and does not require external solutions to keep the nanoparticles in suspension inside the bladder. Furthermore, compared to the use of the cyclic peptide Iso4, or the use of antibodies for targeting the tumor cells, the GNRs@Chit-Apt-Itg overcomes the limitations associated to their high cost of production due to quality control tests and use of animals³⁶.

This study delivers a solution for the recognition of bladder tumors expressing the integrin α5β1. Being the GNRs@Chit-Apt-Itg used as contrast agent for photoacoustic imaging that provides spatial resolution in the range of 50-100 microns¹³, this novel, cost-effective and reproducible nanosystem allows for the detection of small tumor areas, including the tumor margins. Moreover the same system can be applied to GNRs-assisted PTT.

### References

1 Ferlay, J. et al. Cancer incidence and mortality worldwide: sources, methods and major patterns in GLOBOCAN 2012. International journal of cancer 136, E359-386, doi:10.1002/ijc.29210 (2015).
2 Comperat, E. et al. Clinicopathological characteristics of urothelial bladder cancer in patients less than 40 years old. Virchows Arch 466, 589-594, doi: 10.1007/s00428-015-1739-2 (2015).
3 https://uroweb.org/guidelines/non-muscle-invasive-bladder-cancer.
4 Rakesh Heer, R. L., Thenmalar Vadiveloo, Ge Yu, Paramananthan Mariappan, Joanne Cresswell, John McGrath, Ghulam Nabi, Hugh Mostafid, Henry Lazarowicz, John Kelly, Anne Duncan, Steven Penegar, Matt Breckons, Laura Wilson,Emma Clark, Andy Feber, Giovany Orozco-Leal, Zafer Tandogdu, Ernest Taylor, James N'Dow, John Norrie, Craig Ramsay, Stephen Rice, Luke Vale, Graeme MacLennan, Emma Hall. A Randomized Trial of PHOTOdynamic Surgery in Non-Muscle-Invasive Bladder Cancer. NEJM Evidence 1, doi:https://doi.org/10.1056/EVIDoa2200092 (2022).
5 Mossanen, M. & Gore, J. L. The burden of bladder cancer care: direct and indirect costs. Curr Opin Urol 24, 487-491, doi:10.1097/MOU.0000000000000078 (2014).
6 Leal, J., Luengo-Fernandez, R., Sullivan, R. & Witjes, J. A. Economic Burden of Bladder Cancer Across the European Union. Eur Urol 69, 438-447, doi:10.1016/j.eururo.2015.10.024 (2016).
7 Mariotto, A. B., Yabroff, K. R., Shao, Y., Feuer, E. J. & Brown, M. L. Projections of the cost of cancer care in the United States: 2010-2020. J Natl Cancer Inst 103, 117-128, doi:10.1093/jnci/djq495 (2011).
8 Saita, A. et al. Assessing the Feasibility and Accuracy of High-resolution Microultrasound Imaging for Bladder Cancer Detection and Staging. Eur Urol 77, 727-732, doi:10.1016/j.eururo.2019.03.044 (2020).
9 Diana Pietro, L. G., Saita Alberto, Uleri Alessandro, Frego Nicola, Contieri Roberto, Buffi Nicolomaria, Balzarini Luca, D'Orazio Federico, Piergiuseppe Colombo, Elefante Maria Grazia, Lazzeri Massimo, Guazzoni Giorgio, Casale Paolo, Hurle Rodolfo. Head-to-Head Comparison between High-Resolution Microultrasound Imaging and Multiparametric MRI in Detecting and Local Staging of Bladder Cancer: The BUS-MISS Protocol Bladder Cancer 8, 119-127, doi:10.3233BLC-211611 (2022).
10 Babjuk, M. et al. European Association of Urology Guidelines on Non-muscle-invasive Bladder Cancer (TaT1 and Carcinoma In Situ) - 2019 Update. Eur Urol, doi:10.1016/j.eururo.2019.08.016 (2019).
11 Zapala, P. et al. Clinical rationale and safety of restaging transurethral resection in indication-stratified patients with high-risk non-muscle-invasive bladder cancer. World J Surg Oncol 16, 6, doi:10.1186/s12957-018-1310-0 (2018).
12 Alchera, E. et al. Early diagnosis of bladder cancer by photoacoustic imaging of tumor-targeted gold nanorods. Photoacoustics 28, 100400, doi:10.1016/j.pacs.2022.100400 (2022).
13 Steinberg, I. et al. Photoacoustic clinical imaging. Photoacoustics 14, 77-98, doi:10.1016/j.pacs.2019.05.001 (2019).
14 Bryzgunova, O. E. & Laktionov, P. P. Extracellular Nucleic Acids in Urine: Sources, Structure, Diagnostic Potential. Acta Naturae 7, 48-54 (2015).
15 Fechter, P. et al. RNA Aptamers Targeting Integrin alpha5beta1 as Probes for Cyto- and Histofluorescence in Glioblastoma. Mol Ther Nucleic Acids 17, 63-77, doi:10.1016/j.omtn.2019.05.006 (2019).
16 Sun, H. & Zu, Y. Aptamers and their applications in nanomedicine. Small 11, 2352-2364, doi:10.1002/smll.201403073 (2015).
17 Cai, R., Chen, X., Zhang, Y., Wang, X. & Zhou, N. Systematic bio-fabrication of aptamers and their applications in engineering biology. Syst Microbiol Biomanuf 3, 223-245, doi:10.1007/s43393-022-00140-5 (2023).
18 Powell Gray, B. et al. Tunable cytotoxic aptamer-drug conjugates for the treatment of prostate cancer. Proc Natl Acad Sci U S A 115, 4761-4766, doi:10.1073/pnas.1717705115 (2018).
19 Camorani, S. et al. Novel Aptamers Selected on Living Cells for Specific Recognition of Triple-Negative Breast Cancer. iScience 23, 100979, doi:10.1016/j.isci.2020.100979 (2020).
20 Luke, G. P., Nam, S. Y. & Emelianov, S. Y. Optical wavelength selection for improved spectroscopic photoacoustic imaging. Photoacoustics 1, 36-42, doi:10.1016/j.pacs.2013.08.001 (2013).
21 unafold.org/mfold/applications/rna-folding-form.php, h. w.
22 Agnello, L. et al. Optimizing cisplatin delivery to triple-negative breast cancer through novel EGFR aptamer-conjugated polymeric nanovectors. J Exp Clin Cancer Res 40, 239, doi:10.1186/s13046-021-02039-w (2021).
23 Camorani, S., Crescenzi, E., Fedele, M. & Cerchia, L. Oligonucleotide aptamers against tyrosine kinase receptors: Prospect for anticancer applications. Biochim Biophys Acta Rev Cancer 1869, 263-277, doi:10.1016/j.bbcan.2018.03.003 (2018).
24 White, R. et al. Generation of species cross-reactive aptamers using "toggle" SELEX. Mol Ther 4, 567-573, doi:10.1006/mthe.2001.0495 (2001).
25 https://www.malvernpanalytical.com/en/products/measurement-type/zeta-potential.
26 Broberg, A. et al. In-peptide amino acid racemization via inter-residue oxazoline intermediates during acidic hydrolysis. Amino Acids 53, 323-331, doi:10.1007/s00726-021-02951-7 (2021).
27 Alfano, M. et al. A simple and robust nanosystem for photoacoustic imaging of bladder cancer based on alpha5beta1-targeted gold nanorods. J Nanobiotechnology 21, 301, doi:10.1186/s12951-023-02028-5 (2023).
28 Monaco, I. et al. Aptamer Functionalization of Nanosystems for Glioblastoma Targeting through the Blood-Brain Barrier. J Med Chem 60, 4510-4516, doi:10.1021/acs.jmedchem.7b00527 (2017).
29 Ooi, E. H., Popov, V., Alfano, M. & Cheong, J. K. K. Influence of natural convection on gold nanorods-assisted photothermal treatment of bladder cancer in mice. Int J Hyperthermia 37, 634-650, doi:10.1080/02656736.2020.1771437 (2020).
30 Cheong, J. K. et al. A numerical study to investigate the effects of tumour position on the treatment of bladder cancer in mice using gold nanorods assisted photothermal ablation. Comput Biol Med 138, 104881, doi:10.1016/j.compbiomed.2021.104881 (2021).
31 American National Standard for Safe Use of Lasers, A. S. Z.-., NY (2007).
32 Hajipour, M. J. et al. Sex as an important factor in nanomedicine. Nat Commun 12, 2984, doi:10.1038/s41467-021-23230-9 (2021).
33 Uhlig, A. et al. Gender-specific Differences in Recurrence of Non-muscle-invasive Bladder Cancer: A Systematic Review and Meta-analysis. Eur Urol Focus 4, 924-936, doi:10.1016/j.euf2017.08.007 (2018).
34 Pederzoli, F. et al. Sex-specific Alterations in the Urinary and Tissue Microbiome in Therapy-naive Urothelial Bladder Cancer Patients. Eur Urol Oncol 3, 784-788, doi:10.1016/j.euo.2020.04.002 (2020).
35 Aziz, A., Holthof, J., Meyer, S., Schmidt, O. G. & Medina-Sanchez, M. Dual Ultrasound and Photoacoustic Tracking of Magnetically Driven Micromotors: From In Vitro to In Vivo. Adv Healthc Mater 10, e2101077, doi:10.1002/adhm.202101077 (2021).
36 Mascini, M. Aptamers and their applications. Anal Bioanal Chem 390, 987-988, doi:10.1007/s00216-007-1769-y (2008).

## Claims

1. A multifunctional system comprising i) a nucleotide aptamer or a variant thereof that binds to the alpha 5 subunit of integrin and ii) a metal nanoparticle, wherein said aptamer comprises or consists of a nucleotide sequence that is at least 70% identical of 5'-GCGGACGGACAGAGAGUGCAACCUGCCGUGCCGC-3' (SEQ ID No: 1) or functional fragment thereof and optionally comprises at least one modification capable of enhancing nuclease resistance.

2. The multifunctional system according to claim 1 wherein the at least one modification capable of enhancing nuclease resistance is fluorination in the 2'-position of a pyrimidine nucleotide.

3. The multifunctional system according to claim 1 and 2 wherein the aptamer or variant thereof or functional fragments thereof comprises or consists of a nucleotide sequence that is at least 70% identical of: 5'-G(2'F-C)GGA(2'F-C)GGA(2'F-C)AGAGAG(2'F-U)G(2'F-C)AA(2'F-C)(2'F-C)(2'F-U)G(2'F-C)(2'F-C)G(2'F-U)G(2'F-C)(2'F-C)G(2'F-C)-3' (SEQ ID No:2).

4. The multifunctional system according to any one of previous claims wherein the aptamer or variant thereof or functional fragments thereof is functionalized at its 5' end with a thiol group, preferably with a C6-C12 aliphatic-thio molecule, preferably wherein the aptamer comprises or consists of a nucleotide sequence that is at least 70% identical of SEQ ID No: 2 or variant thereof or functional fragment thereof and is functionalized with a C6-thiol group at its 5'end.

5. The multifunctional system according to any one of previous claims wherein the metal nanoparticle is made of gold, silver, or hybrid gold/silver, preferably gold and the metal nanoparticle has photoacoustic properties, preferably wherein the metal nanoparticle is chosen among nanosphere, nanorod, nanostar, nanocage, nanoprism or nanoshell or nanowire, nanoplate, hallow shell, preferably the metal nanoparticle is gold nanorod with a size ranging from 10 to 200 nm in length, and 2 to 50 nm in width, more preferably ranging from 20 to 100 nm in length and from 10 to 25 nm in width and the preferred aspect ratio ranges between 1.2 and 15, more preferably between from 3 and 7.

6. The multifunctional system according to any one of previous claims wherein the metal nanoparticle is coated with a polymer functionalized with thiol groups and -NH groups, said polymer preferably being selected among thiolated chitosan, thiolated and aminated alginic acid, thiolated and aminated hyaluronic acid, or a protein preferably selected from the group consisting of albumin and gelatin, or a synthetic thiolated and aminated polymer, preferably α-thio-ω-amino polyethylene glycols.

7. The multifunctional system according to claim 6 wherein the polymer functionalized with thiol groups and -NH groups is thiolated chitosan preferably having an average molecular weight ranging from 0.5 kDa to 1000 kDa, more preferably ranging from 50 kDa to 200 kDa, and an average deacetylation degree ranging from 75% to 100%, more preferably ranging from 85% to 95%.

8. The multifunctional system according to any one of claims 6 and 7, wherein said thiol groups and
- NH groups of the polymer are linked via a crosslinker to the aptamer that binds to the alpha 5 subunit of integrin and wherein:
- the crosslinker bears at least one functional group able to bind to an amino group, said functional group being preferably selected from the group consisting of N-hydroxysuccinimidyl ester group (NHS ester), an isocyanate group (-NCO), an isothiocyanate group (-NCS), a sulfo-N-hydroxysuccinimidyl ester group (sulfo-NHS ester), a maleimide group or a carboxylic acid; and/or
- the cross linker bears at least one functional group able to bind a thiol group, said functional group being preferably selected from the group consisting of: a maleimide group, a terminal vinyl group or a terminal alkyne group; and/or
- the cross linker bears at least one functional group able to bind an alkyne such as an azide; and/or
- the cross linker bear at least one functional group able to bind an azide, such as an alkyne.

9. The multifunctional system according to claim 8 wherein the crosslinker is maleimido-PEG₁₂-NHS ester.

10. The multifunctional system according to any one of the preceding claims wherein:
- the aptamer has SEQ ID No:2 and is preferably functionalized at its 5'end with a C6-thiol group;
- the metal nanoparticle is gold nanorod;
- the functionalized polymer is thiolated chitosan;
- the crosslinker is maleimido-PEG₁₂-NHS ester.

11. A composition comprising the multifunctional system according to any of the preceding claims or a kit comprising single use vials containing the multifunctional system according to any one of the preceding claims, said composition and kit further comprising at least one of following solvent: water; physiologic solution; Dulbecco's Modified Eagle Medium (DMEM); Dulbecco's phosphate-buffered saline (DPBS), HEPES buffer, TRIS buffer, PIPES buffer each containing metal divalent ions such as Ca²⁺, Mg²⁺.

12. The composition and kit according to claim 11 comprising at least one or more antitumoral agents preferably chosen among a chemotherapeutic agent, an immunomodulator, an immune cell.

13. The multifunctional system according to claims 1-10 or the composition and kit according to claim 11-12 for medical use, preferably for use in a method of diagnosis and/or treatment in vivo.

14. The multifunctional system or composition or kit according to claim 13 for use in the diagnosis and/or treatment of solid tumors.

15. The multifunctional system or composition or kit according to claim 14 wherein the tumor is selected among urothelial, bladder, gastroesophageal, colorectal, pancreatic, ovarian, lung, cervix, breast and renal cancer, brain tumors, hollow organs cancer and hepatocellular carcinoma.

16. The multifunctional system or composition or kit according to any of claims 13-15 for use in the photothermal therapy of solid tumors, preferentially bladder cancer.

17. An in-vitro diagnostic method for detecting cancer from a biological sample of a test subject, said method comprising the following steps:
i) contacting said biological sample with at least one multifunctional system according to claims 1-10 or with the composition or kit according to claim 11-12, and
j) photoacoustic or ultrasound visualization of the test sample, and
k) detection of the presence of alpha 5 beta 1 integrin, and
l) evaluation by comparison with a reference sample.

18. An aptamer that binds to the alpha 5 subunit of integrin comprising or consisting of a nucleotide sequence that is at least 70% identical of 5'-GCGGACGGACAGAGAGUGCAACCUGCCGUGCCGC-3' (SEQ ID No: 1) or a variant thereof or a functional fragment thereof and has at least one modification capable of enhancing nuclease resistance, preferably wherein the at least one modification capable of enhancing nuclease resistance is fluorination in the 2'-position of a pyrimidine nucleotide, preferably wherein the aptamer comprises or consists of a nucleotide sequence that is at least 70% identical of: 5'-G(2'F-C)GGA(2'F-C)GGA(2'F-C)AGAGAG(2'F-U)G(2'F-C)AA(2'F-C)(2'F-C)(2'F-U)G(2'F-C)(2'F-C)G(2'F-U)G(2'F-C)(2'F-C)G(2'F-C)-3' (SEQ ID No:2) or a variant thereof or a functional fragment thereof.
